# EUROPÄISCHE PATENTSCHRIFT

(11) **EP 0 628 540 B1**
(45) Veröffentlichungstag und Bekanntmachung des Hinweises auf die Patenterteilung: **20.01.1999**
(21) Anmeldenummer: 94108040.0
(22) Anmeldetag: 25.05.1994
(51) Int. Cl.: C07C 251/48, C07C 323/60, C07C 323/62, C07D 307/82, C07D 317/46, C07D 319/08, C07D 319/20, A01N 37/50

(54) **2-Oximino-2-phenyl-acetamide**
2-Oximino-2-phenyl-acetamides
2-Oximino-2-phényl acétamides

(30) Priorität: 07.06.1993 DE 4318917
(43) Veröffentlichungstag der Anmeldung: 14.12.1994
(73) Patentinhaber: BAYER AG, 51368 Leverkusen (DE)
(72) Erfinder: Gerdes, Peter, Dr., D-52080 Aachen (DE); Gayer, Herbert, Dr., D-40789 Monheim (DE); Dutzmann, Stefan, Dr., D-40721 Hilden (DE); Dehne, Heinz-Wilhelm, Dr., D-40789 Monheim (DE)

(56) Entgegenhaltungen:
- EP-A- 0 398 692
- EP-A- 0 535 928
- EP-A- 0 579 124
- CHEMICAL ABSTRACTS, vol. 118, no. 7, 1993, Columbus, Ohio, US; abstract no. 59429e, & RN = 145451-01-0 & JP-A-4 182 461 (SHIONOGI AND CO., LTD.) 30. Juni 1992

## Beschreibung

Die Erfindung betrifft neue 2-Oximino-2-phenyl-acetamide, ein Verfahren zu ihrer Herstellung und ihre Verwendung als Schädlingsbekämpfungsmittel.

Es ist bekannt, daß bestimmte 2-Methoximino-2-phenyl-essigsäureester fungizide Eigenschaften besitzen (vgl. EP-OS 0 400 417 JP-4 182 461). So läßt sich zum Beispiel 2-Methoximino-2-[2-(2-methylphenoxymethyl)-phenyl]-essigsäuremethylester zur Bekämpfung von Pilzen verwenden. Die Wirksamkeit dieses Stoffes ist aber insbesondere bei niedrigen Aufwandmengen nicht in allen Anwendungsgebieten völlig zufriedenstellend.

Es wurden nun neue 2-Oximino-2-phenyl-acetamide der Formel 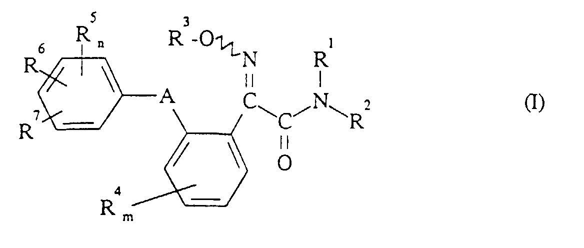 in welcher
- R¹: für Wasserstoff, geradkettiges oder verzweigtes Alkyl mit 1 bis 8 Kohlenstoffatomen oder geradkettiges oder verzweigtes Alkoxy mit 1 bis 8 Kohlenstoffatomen steht,
- R²: für Wasserstoff, geradkettiges oder verzweigtes Alkyl mit 1 bis 8 Kohlenstoffatomen oder geradkettiges oder verzweigtes Alkoxy mit 1 bis 8 Kohlenstoffatomen steht,
- R³: für Wasserstoff, geradkettiges oder verzweigtes Alkyl mit 1 bis 8 Kohlenstoffatomen oder geradkettiges oder verzweigtes Alkoxy mit 1 bis 8 Kohlenstoffatomen steht,
- R⁴: für Halogen, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, ALkylsulfonyloxy, Hydroximinoalkyl oder Alkoximinoalkyl mit jeweils 1 bis 6 Kohlenstoffatomen in den einzelnen Alkyl- bzw. Alkoxyteilen, außerdem für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
ferner für gegebenenfalls einfach bis dreifach, gleichartig oder verschieden durch Halogen und/oder Alkyl mit 1 bis 4 Kohlenstoffatomen substituiertes 3- bis 7-gliedriges Heterocyclyl mit 2 bis 6 Kohlenstoffatomen und 1 bis 3 gleichen oder verschiedenen Heteroatomen, wie Stickstoff, Sauerstoff und/oder Schwefel steht,
und weiterhin
für Phenyl, Phenoxy, Benzyl, Benzyloxy, Phenylethyl oder Phenylethyloxy steht, wobei jeder dieser Reste im Phenylteil einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen,
- R⁵: für Halogen, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 6 Kohlenstoffatomen in den einzelnen Alkyl- bzw. Alkoxy-teilen, außerdem für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
ferner für gegebenenfalls einfach bis dreifach, gleichartig oder verschieden durch Halogen und/oder Alkyl mit 1 bis 4 Kohlenstoffatomen substituiertes 3- bis 7-gliedriges Heterocyclyl mit 2 bis 6 Kohlenstoffatomen und 1 bis 3 gleichen oder verschiedenen Heteroatomen, wie Stickstoff, Sauerstoff und/oder
Schwefel steht,
und weiterhin,
für Phenyl, Phenoxy, Benzyl, Benzyloxy, Phenylethyl oder Phenylethyloxy steht, wobei jeder dieser Reste im Phenylteil einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen,
- m: für die Zahlen 0, 1, 2, 3 oder 4 steht, wobei R⁴ für gleiche oder verschiedene Reste stehen kann, wenn m für 2, 3 oder 4 steht.
- n: für die Zahlen 1, 2 oder 3 steht, wobei R⁵ für gleiche oder verschiedene Reste stehen kann. wenn n für 2, 3 oder 4 steht.
- A: für eine Gruppierung der Formel -CH₂-O-, -O-CH₂-. -CH₂-S- oder -S-CH₂- steht,
- R⁶: für Wasserstoff, Halogen, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 6 Kohlenstoffatomen in den einzelnen Alkyl-bzw. Alkoxyteilen, außerdem für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
ferner für gegebenenfalls einfach bis dreifach, gleichartig oder verschieden durch Halogen und/oder Alkyl mit 1 bis 4 Kohlenstoffatomen substituiertes 3- bis 7-gliedriges Heterocyclyl mit 2 bis 6 Kohlenstoffatomen und 1 bis 3 gleichen oder verschiedenen Heteroatomen, wie Stickstoff, Sauerstoff und/oder Schwefel steht,
und weiterhin
für Phenyl, Phenoxy, Benzyl, Benzyloxy, Phenylethyl oder Phenylethyloxy steht, wobei jeder dieser Reste im Phenylteil einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen und oder geradkettiges oder verzweigtes Halogenalkoxy mit 1 bis 4 Kohlenstoffatomen und 1 bis 9 gleichen oder verschiedenen Halogenatomen;
- R⁷: für jeweils geradkettiges oder verzweigtes Halogenalkoxy oder Halogenalkylthio mit jeweils 1 bis 8 Kohlenstoffatomen und 1 bis 17 gleichen oder verschiedenen Halogenatomen - insbesondere Fluor, Chlor, Brom und/oder Iod steht,
- R⁶ und R⁷: außerdem auch gemeinsam vorzugsweise für einen gegebenenfalls einfach bis sechsfach, gleichartig oder verschieden durch Halogen, geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - substituierten zweifach verknüpften Alkandiylrest mit 1 bis 5 Kohlenstoffatomen stehen, der 1 bis 3 Sauerstoff- und/oder Schwefelatome enthält,
gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Die Erfindung betrifft sowohl die reinen Isomeren als auch Isomerengemische.

Weiterhin wurde gefunden daß man 2-Oximino-2-phenyl-acetamide der Formel (I) erhält, wenn man 2-Oximino-2-phenyl-essigsäurester der Formel 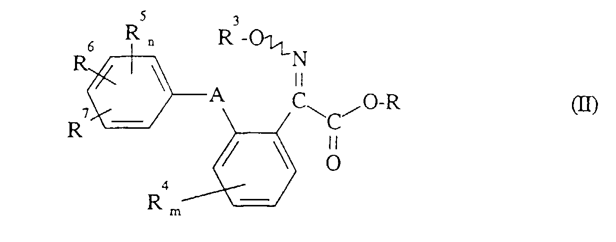 in welcher
- R³, R⁴, R⁵, R⁶, R⁷, A, m und n: die oben angegebenen Bedeutungen haben und
- R: für Alkyl steht,
mit Ammoniak oder Aminen der Formel 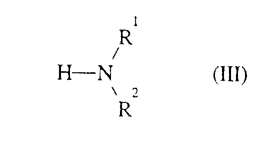 in welcher
- R¹ und R²: die oben angegebenen Bedeutungen haben,
gegebenenfalls in Gegenwart eines Verdünnungsmittels und gegebenenfalls in Gegenwart eines Säurebindemittels umsetzt.

Schließlich wurde gefunden, daß die neuen 2-Oximino-2-phenyl-acetamide der Formel (I) sehr gut zur Schädlingsbekämpfung geeignet sind.

Überraschenderweise zeigen die erfindungsgemäßen 2-Oximino-2-phenyl-acetamide der Formel (I) gegen pflanzenschädigende Mikroorganismen eine erheblich bessere Wirksamkeit als 2-Methoximino-2-[2-(2-methyl-phenoxymethyl)-phenyl]-essigsäuremethylester, welches ein konstitutionell ähnlicher, vorbekannter Wirkstoff gleicher Wirkungsrichtung ist.

Die erfindungsgemäßen 2-Oximino-2-phenyl-acetamide sind durch die Formel (I) allgemein definiert.
- R¹: steht besonders bevorzugt für Wasserstoff, geradkettiges oder verzweigtes Alkyl mit 1 bis 6 Kohlenstoffatomen oder geradkettiges oder verzweigtes Alkoxy mit 1 bis 6 Kohlenstoffatomen.
- R²: steht besonders bevorzugt für Wasserstoff, geradkettiges oder verzweigtes Alkyl mit 1 bis 6 Kohlenstoffatomen oder geradkettiges oder verzweigtes Alkoxy mit 1 bis 6 Kohlenstoffatomen.
- R³: steht besonders bevorzugt für Wasserstoff, geradkettiges oder verzweigtes Alkyl mit 1 bis 6 Kohlenstoffatomen oder geradkettiges oder verzweigtes Alkoxy mit 1 bis 6 Kohlenstoffatomen.
- R⁴: steht besonders bevorzugt für Fluor, Chlor, Brom, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Fluor-, Chlor- und/oder Bromatomen, jeweils geradkettiges oder verzweigtes Halogenalkenyl oder Halogenalkenyloxy mit jeweils 2 bis 4 Kohlenstoffatomen und 1 bis 9 Fluor-, Chlor- und/oder Bromatomen, jeweils geradkettiges oder verzweigtes N-Alkylamino, Dialkylamino, Alkylcarbonyl, Alkylcarbonyloxy. Alkoxycarbonyl, Alkylsulfonyloxy, Hydroximinoalkyl oder Alkoximinoalkyl mit jeweils 1 bis 4 Kohlenstoffatomen in den einzelnen Alkyl- bzw. Alkoxyteilen, außerdem für gegebenenfalls einfach bis dreifach, gleichartig oder verschieden durch Fluor, Chlor, Methyl und/oder Ethyl substituiertes Cycloalkyl mit 3 bis 6 Kohlenstoffatomen,
ferner für gegebenenfalls einfach bis dreifach, gleichartig oder verschieden durch Fluor, Chlor, Methyl und/oder Ethyl substituiertes, 3- bis 7-gliedriges Heterocyclyl mit 2 bis 5 Kohlenstoffatomen und 1 bis 3 gleichen oder verschiedenen Heteroatomen, wie Stickstoff, Sauerstoff und/oder Schwefel,
und weiterhin
für Phenyl, Phenoxy, Benzyl, Benzyloxy, Phenylethyl oder Phenylethyloxy, wobei jeder dieser Reste im Phenylteil einfach bis fünffach, gleichartig oder verschieden substituiert sein kann durch Fluor, Chlor, Brom, geradkettiges oder verzweigtes Alkyl mit 1 bis 3 Kohlenstoffatomen, geradkettiges oder verzweigtes Halogenalkyl mit 1 bis 3 Kohlenstoffatomen und 1 bis 7 Fluor-, Chlor- und/oder Bromatomen, geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 Fluor-, Chlor- und/oder Bromatomen.
- R⁵: steht besonders bevorzugt für Fluor, Chlor, Brom, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Fluor-, Chlor- und/oder Bromatomen, jeweils geradkettiges oder verzweigtes Halogenalkenyl oder Halogenalkenyloxy mit jeweils 2 bis 4 Kohlenstoffatomen und 1 bis 9 Fluor-, Chlor- und/oder Bromatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 4 Kohlenstoffatomen in den einzelnen Alkyl- bzw. Alkoxyteilen, außerdem für gegebenenfalls einfach bis dreifach, gleichartig oder verschieden durch Fluor, Chlor, Methyl und/oder Ethyl substituiertes Cycloalkyl mit 3 bis 6 Kohlenstoffatomen,
ferner für gegebenenfalls einfach bis dreifach, gleichartig oder verschieden durch Fluor, Chlor, Methyl und/oder Ethyl substituiertes, 3- bis 7-gliedriges Heterocyclyl mit 2 bis 5 Kohlenstoffatomen und 1 bis 3 gleichen oder verschiedenen Heteroatomen, wie Stickstoff, Sauerstoff und/oder Schwefel,
und weiterhin
für Phenyl, Phenoxy, Benzyl, Benzyloxy, Phenylethyl oder Phenylethyloxy, wobei jeder dieser Reste im Phenylteil einfach bis fünffach, gleichartig oder verschieden substituiert sein kann durch Fluor, Chlor, Brom, geradkettiges oder verzweigtes Alkyl mit 1 bis 3 Kohlenstoffatomen, geradkettiges oder verzweigtes Halogenalkyl mit 1 bis 3 Kohlenstoffatomen und 1 bis 7 Fluor-, Chlor- und/oder Bromatomen, geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 Fluor-, Chlor- und/oder Bromatomen.
- m: steht besonders bevorzugt für die Zahlen 0, 1, 2 oder 3, wobei R⁴ für gleiche oder verschiedene Reste stehen kann, wenn m für 2 oder 3 steht.
- n: steht besonders bevorzugt für die Zahlen 1, 2 oder 3, wobei R⁵ für gleiche oder verschiedene Reste stehen kann, wenn n für 2 oder 3 steht.
- A: steht besonders bevorzugt für eine Gruppierung der Formel -CH₂-O-, -O-CH₂-, -CH₂-S- oder -S-CH₂-,
- R⁶: steht besonders bevorzugt für Wasserstoff, Fluor, Chlor, Brom. Cyano, Nitro. Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Fluor-, Chlor- und/oder Bromatomen, jeweils geradkettiges oder verzweigtes Halogenalkenyl oder Halogenalkenyloxy mit jeweils 2 bis 4 Kohlenstoffatomen und 1 bis 9 Fluor-, Chlor- und/oder Bromatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 4 Kohlenstoffatomen in den einzelnen Alkyl-bzw. Alkoxyteilen,
außerdem für gegebenenfalls einfach bis dreifach, gleichartig oder verschieden durch Fluor, Chlor, Methyl und/oder Ethyl substituiertes Cycloalkyl mit 3 bis 6 Kohlenstoffatomen,
ferner für gegebenenfalls einfach bis dreifach, gleichartig oder verschieden durch Fluor, Chlor, Methyl und/oder Ethyl substituiertes, 3- bis 7-gliedriges Heterocyclyl mit 2 bis 5 Kohlenstoffatomen und 1 bis 3 gleichen oder verschiedenen Heteroatomen, wie Stickstoff, Sauerstoff und/oder Schwefel,
und weiterhin
für Phenyl, Phenoxy, Benzyl, Benzyloxy, Phenylethyl oder Phenylethyloxy, wobei jeder dieser Reste im Phenylteil einfach bis fünffach, gleichartig oder verschieden substituiert sein kann durch Fluor, Chlor, Brom, geradkettiges oder verzweigtes Alkyl mit 1 bis 3 Kohlenstoffatomen, geradkettiges oder verzweigtes Halogenalkyl mit 1 bis 3 Kohlenstoffatomen und 1 bis 7 Fluor-, Chlor- und/oder Bromatomen, geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 Fluor-, Chlor- und/oder Bromatomen.
- R⁷: steht besonders bevorzugt für jeweils geradkettiges oder verzweigtes Halogenalkoxy oder Halogenalkylthio mit jeweils 1 bis 4 Kohlenstoffatomen und 1 bis 9 Fluor-, Chlor- und/oder Bromatomen.
- R⁶ und R⁷: stehen außerdem auch gemeinsam besonders bevorzugt für einen gegebenenfalls einfach bis sechsfach, gleichartig oder verschieden durch Fluor, Chlor, Brom, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 Fluor-, Chlor- und/oder Bromatomen substituierten zweifach verknüpften Alkandiylrest mit 1 bis 4 Kohlenstoffatomen, der 1 oder 2 Sauerstoff- und/oder Schwefelatome enthält.
- R¹: steht ganz besonders bevorzugt für Wasserstoff, geradkettiges oder verzweigtes Alkyl mit 1 bis 4 Kohlenstoffatomen oder für geradkettiges oder verzweigtes Alkoxy mit 1 bis 4 Kohlenstoffatomen.
- R²: steht ganz besonders bevorzugt für Wasserstoff, geradkettiges oder verzweigtes Alkyl mit 1 bis 4 Kohlenstoffatomen oder für geradkettiges oder verzweigtes Alkoxy mit 1 bis 4 Kohlenstoffatomen.
- R³: steht ganz besonders bevorzugt für Wasserstoff, geradkettiges oder verzweigtes Alkyl mit 1 bis 4 Kohlenstoffatomen oder für geradkettiges oder verzweigtes Alkoxy mit 1 bis 4 Kohlenstoffatomen.
- R⁴: steht ganz besonders bevorzugt für Fluor, Chlor, Brom, Cyano, Nitro, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Cyclopropyl, Cyclopentyl, Cyclohexyl, 1-Pyrrolidinyl, 1-Piperidinyl, 1-Perhydroazepinyl, 4-Morpholinyl oder für Phenyl. Phenoxy, Benzyl. Benzyloxy, Phenylethyl oder Phenylethyloxy, wobei jeder der sechs letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy.
- R⁵: steht ganz besonders bevorzugt für Fluor, Chlor, Brom, Cyano. Nitro, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Cyclopropyl, Cyclopentyl, Cyclohexyl, 1-Pyrrolidinyl, 1-Piperidinyl, 1-Perhydroazepinyl, 4-Morpholinyl oder für Phenyl, Phenoxy, Benzyl, Benzyloxy, Phenylethyl oder Phenylethyloxy, wobei jeder der sechs letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy.
- m: steht ganz besonders bevorzugt für die Zahlen 0, 1 oder 2, wobei R⁴ für gleiche oder verschiedene Reste stehen kann, wenn m für 2 steht.
- n: steht ganz besonders bevorzugt für die Zahlen 1 oder 2, wobei R⁵ für gleiche oder verschiedene Reste stehen kann, wenn n für 2 steht.
- A: steht ganz besonders bevorzugt für eine Gruppierung der Formel -CH₂-O-, -O-CH₂-, -CH₂-S- oder -S-CH₂-.
- R⁶: steht ganz besonders bevorzugt für Wasserstoff, Fluor, Chlor, Brom, Cyano, Nitro, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl. Allyl, Butenyl. Allyloxy, Butenyloxy, Trifluormethyl. Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für Phenyl, Phenoxy, Benzyl, Benzyloxy, Phenylethyl oder Phenylethyloxy, wobei jeder der sechs letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy.
- R⁷: steht ganz besonders bevorzugt für geradkettiges oder verzweigtes Halogenalkoxy mit 1 bis 3 Kohlenstoffatomen und 1 bis 7 Fluor-, Chlor- und/oder Bromatomen oder für geradkettiges oder verzweigtes Halogenalkylthio mit 1 bis 3 Kohlenstoffatomen und 1 bis 7 Fluor-, Chlor- und/oder Bromatomen.
- R⁶ und R⁷: stehen außerdem gemeinsam ganz besonders bevorzugt für einen gegebenenfalls einfach bis vierfach, gleich oder verschieden durch Fluor, Chlor, Brom, Methyl, Ethyl und/oder Trifluormethyl substituierten zweifach verknüpften Alkandiylrest mit 1 bis 3 Kohlenstoffatomen, der 1 bis 2 Sauerstoff-oder Schwefelatome enthält.

Im einzelnen seien außer den bei den Herstellungsbeispielen genannten Verbindungen die folgenden 2-Oximino-2-phenyl-acetamide der Formel (I) genannt:

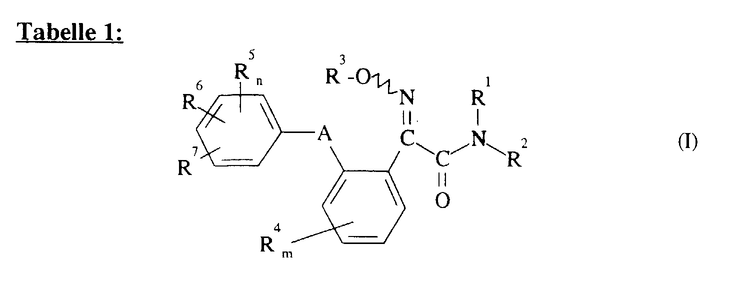

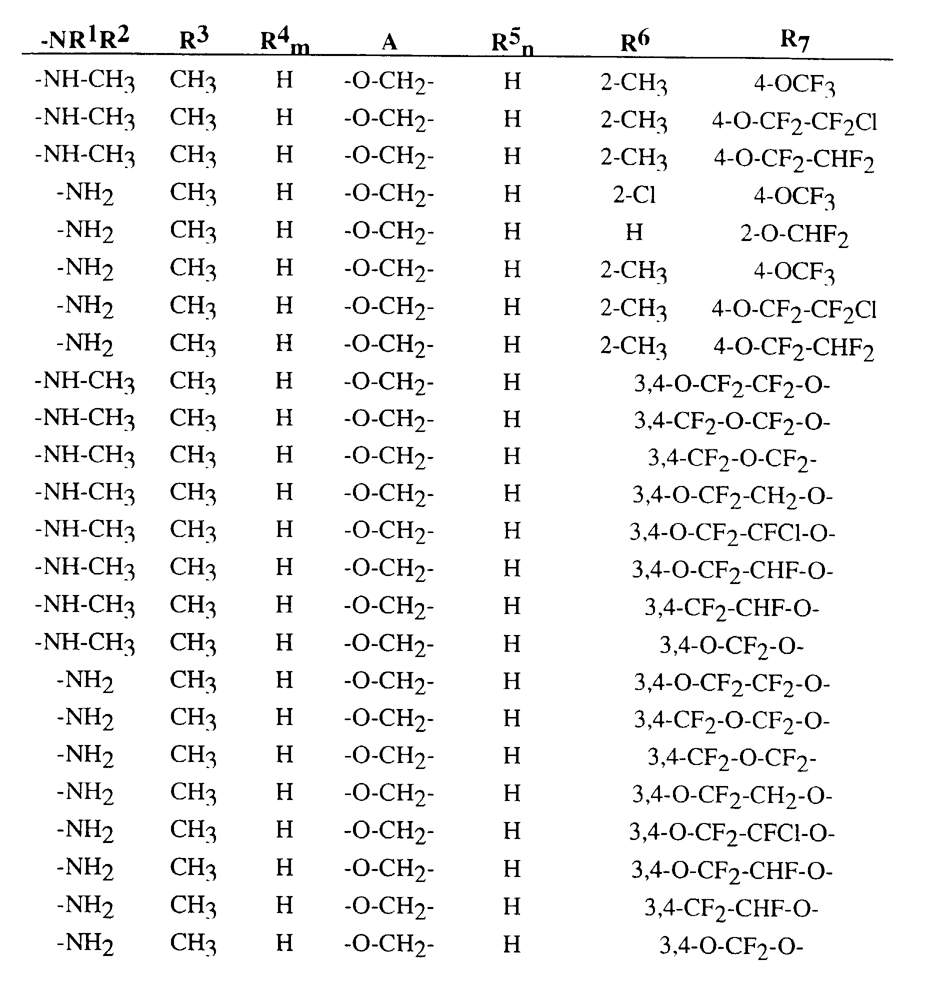

Verwendet man beispielsweise 2-[2-(2-Chlor-4-trifluormethoxy-phenoxymethyl)]-phenyl-2-methoximino-essigsäuremethylester und Methylamin als Ausgangsstoffe, so läßt sich der Verlauf des erfindungsgemäßen Verfahrens durch das folgende Formelschema veranschaulichen:

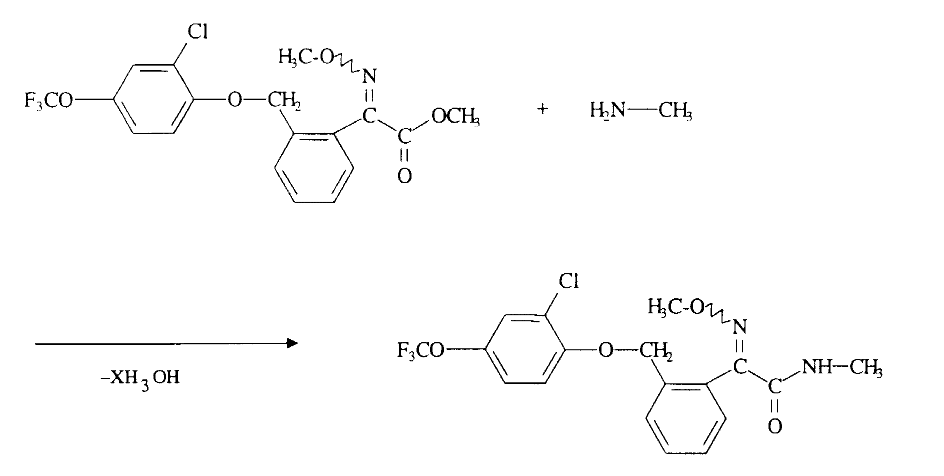

Die zur Durchführung des erfindungsgemäßen Verfahrens als Ausgangsverbindungen benötigten 2-Oximino-2-phenyl-essigsäurester sind durch die Formel (II) allgemein definiert. In dieser Formel (II) stehen R³, R⁴, R⁵, R⁶, R⁷, A, m und n vorzugsweise für diejenigen Reste und Indices, die bereits im Zusammenhang mit der Beschreibung der erfindungsgemäßen Verbindungen der Formel (I) als bevorzugt für diese Substituenten und diese Indices genannt wurden. R steht vorzugsweise für geradkettiges oder verzweigtes Alkyl mit 1 bis 4 Kohlenstoffatomen, insbesondere für Methyl oder Ethyl.

2-Oximino-2-phenyl-essigsäurester der Formel (II) sind bekannt oder lassen sich nach prinzipiell bekannten Verfahren herstellen (vgl. z.B. EP-OS 0 253 213; EP-OS 0 398 692; EP-OS 0 400 417 und EP-OS 0 477 631). So erhält man 2-Oximino-2-phenylessigsäureester der Formel (II), indem man (Thio)Phenole der Formel 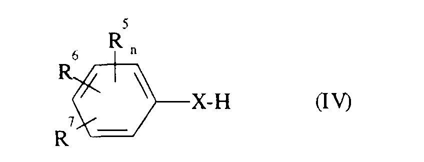 in welcher
- R⁵, R⁶, R⁷ und n: die oben angegebene Bedeutung haben und
- X: für Sauerstoff oder Schwefel steht,
mit 2-(2-Halogenmethylphenyl)-2-methoximinoessigsäureestern der Formel 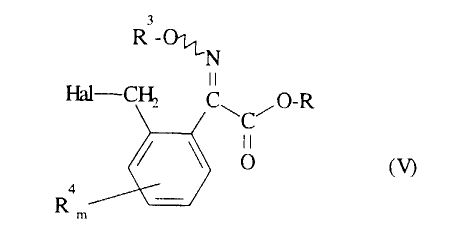 in welcher
- R, R³, R⁴ und m: die oben angegebene Bedeutung haben und
- X: für Halogen steht,
gegebenenfalls in Gegenwart eines Verdünnungsmittels, wie beispielsweise N,N-Dimethylformamid, und gegebenenfalls in Gegenwart eines Säurebindemittels, wie beispielsweise Natriumhydrid, bei Temperaturen zwischen -20°C und +80°C umsetzt.

(Thio)Phenole der Formel (IV) sind allgemein bekannte Verbindungen der organischen Chemie oder erhältlich in Analogie zu bekannten Verfahren (vergl. DE-OS 38 36 175).

2-(2-Halogenmethylphenyl)-2-methoximinoessigsäureester der Formel (V) sind ebenfalls bekannt oder erhältlich in Analogie zu bekannten Verfahren (vergl. EP-OS 0 254 426).

Die zur Durchführung des erfindungsgemäßen Verfahrens weiterhin als Ausgangsstoffe benötigten Amine bzw. Ammoniak sind durch die Formel (III) allgemein definiert. In dieser Formel (III) stehen R¹ und R² vorzugsweise für diejenigen Reste, die bereits im Zusammenhang mit der Beschreibung der erfindungsgemäßen Verbindungen der Formel (I) als bevorzugt für diese Substituenten genannt wurden.

Die Amine der Formel (III) und Ammoniak sind allgemein bekannte Verbindungen der organischen Chemie.

Als Verdünnungsmittel zur Durchführung des erfindungsgemäßen Verfahrens kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren wird vorzugsweise in Gegenwart eines Säurebindemittels durchgeführt. Als solche kommen alle üblichen anorganischen oder organischen Basen infrage. Vorzugsweise verwendbar sind Erdalkali- oder Alkalimetallhydride, -hydroxide, -amide, -alkoholate, -acetate, -carbonate oder -hydrogencarbonate sowie Ammoniumverbindungen, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (III) in einem entsprechenden Überschuß gleichzeitig als Säurebindemittel einzusetzen.

Die Reaktionstemperaturen können bei der Durchführung des erfindungsgemäßen Verfahrens in einem größeren Bereich variiert werden. Im allgemeinen arbeitet man bei Temperaturen zwischen -30°C und +150°C, vorzugsweise bei Temperaturen zwischen -20°C und +120°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an 2-Oximino-2-phenyl-essigsäurester der Formel (II) im allgemeinen 1,0 bis 3,0 Mol, vorzugsweise 1,0 bis 1,5 Mol an Amin der Formel (III) und gegebenenfalls 0,1 bis 3,0 Mol, vorzugsweise 0,5 bis 1,5 Mol an Säurebindemittel ein. Dabei ist es in einer besonderen Durchführungsform auch möglich, die als Ausgangsprodukte verwendeten 2-Oximino-2-phenyl-essigsäurester der Formel (II) in einer vorgelagerten Reaktion im Reaktionsgefäß herzustellen und anschließend ohne Isolierung direkt in einer sogenannten "Eintopfreaktion" nach dem erfindungsgemäßen Verfahren weiter umzusetzen. Die Reaktionsdurchführung, Aufarbeitung und Isolierung der Reaktionsprodukte erfolgt jeweils in üblicher Weise.

Die Reinigung der Endprodukte der Formel (I) erfolgt mit Hilfe üblicher Verfahren, beispielsweise durch Säulenchromatographie oder durch Umkristallisieren. Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe weisen eine starke Wirkung gegen Schädlinge auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes. Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger der Tomatenbraunfäule (Phytophthora infestans) oder gegen den Erreger der falschen Rebenmehltaues (Plasmopara viticola) oder gegen den Erreger der Sclerotinia-Fäule an Bohnen (Sclerotinia sclerotiorum) oder gegen den Erreger des Apfelschorfes (Venturia inaequalis) oder gegen den Erreger des echten Gurkenmehltaues (Sphaerotheca fuliginea) oder gegen den Erreger des Apfelmehltaues (Podosphaera leucotricha) oder gegen den Erreger des echten Rebenmehltaues (Uncinula necator) oder zur Bekämpfung von Getreidekrankheiten wie beispielsweise gegen den Erreger der Halmbruchkrankheit des Weizens (Pseudocercosporella herpotrichoides) oder gegen den Erreger des echten Getreidemehltaues (Erysiphe graminis) oder gegen den Erreger der Netzfleckenkrankheit der Gerste (Pyrenophora teres) oder gegen den Erreger der Braunfleckenkrankheit der Gerste oder des Weizens (Cochliobolus sativus) oder gegen den Erreger der Braunspelzigkeit des Weizens (Septoria nodorum) oder gegen Fusariumarten oder zur Bekämpfung von Reiskrankheiten, wie beispielsweise gegen den Erreger der Reisfleckenkrankheit (Pyricularia oryzae) eingesetzt werden. Daneben besitzen die erfindungsgemäßen Wirkstoffe eine gute in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die Herstellung und die Verwendung der erfindungsgemäßen Stoffe gehen aus den folgenden Beispielen hervor.

### Herstellungsbeispiele:

### Beispiel 1:

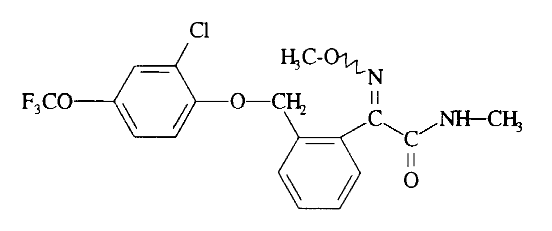

Zu einer Lösung von 4,2 g (0,02 Mol) 2-Chlor-4-trifluormethoxyphenol und 8,1 g (0,02 Mol) 2-(2-Brommethylphenyl)-2-methoximino-essigsäuremethylester in 50 ml N,N-Dimethylformamid gibt man bei 0°C unter Rühren 0,6 g (0,02 Mol) Natriumhydrid (80-prozentig), laßt den Reaktionsansatz anschließend langsam auf Raumtemperatur kommen und rührt 4 Stunden bei dieser Temperatur. Zur Aufarbeitung gibt man 50 ml Wasser zu und extrahiert mit Essigsäureethylester. Die organische Phase wird getrocknet, unter vermindertem Druck eingeengt und der Rückstand wird in 50 ml Methanol aufgenommen. Zu der entstehenden Lösung gibt man 6,2 ml (0,062 Mol) Methylaminlösung (30-prozentig in Wasser), rührt anschließend 16 Stunden bei Raumtemperatur und entfernt dann das Lösungsmittel unter vermindertem Druck. Der Rückstand wird durch Chromatographie an Kieselgel (Laufmittel: Essigester/Hexan) gereinigt.

Man erhält 2,5 g (48 % der Theorie) an 2-[2-(2-Chlor-4-trifluormethoxy-phenoxymethyl)-phenyl]-2-methoximino-essigsäure-N-methylamid als Öl.
- ¹H-NMR (CDCl₃/Tetramethylsilan): d =: 2.88 (d, 3H); 3,93 (s, 3H); 5,06 (s, 2H); 6,75 (s, 1H); 7,2 (m, 7H) ppm
In entsprechender Weise und gemäß den allgemeinen Angaben zur Herstellung erhält man die folgenden 2-Oximino-2-phenyl-acetamide der Formel (I)

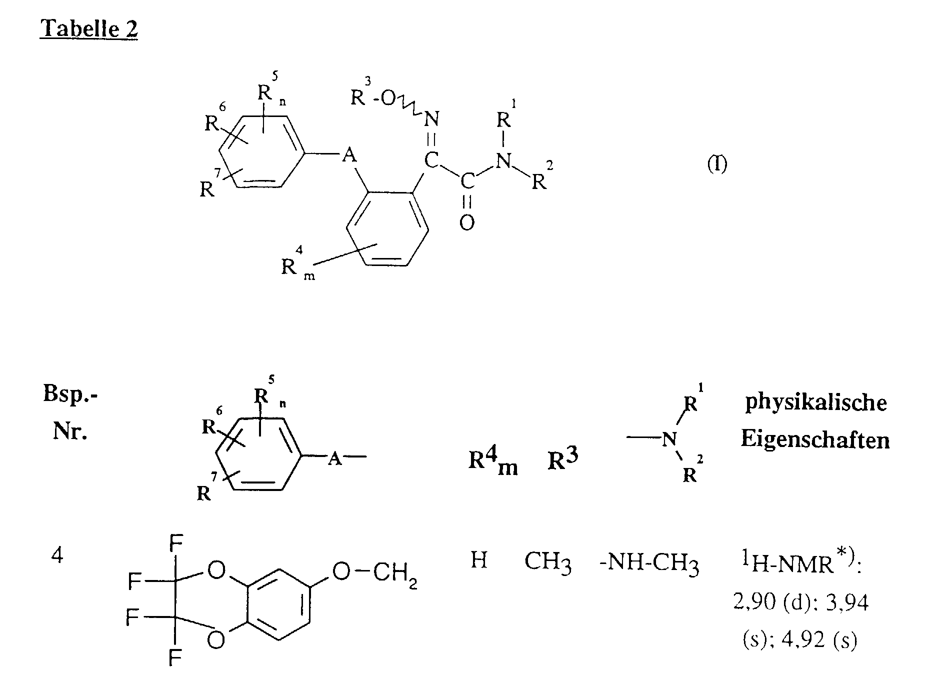

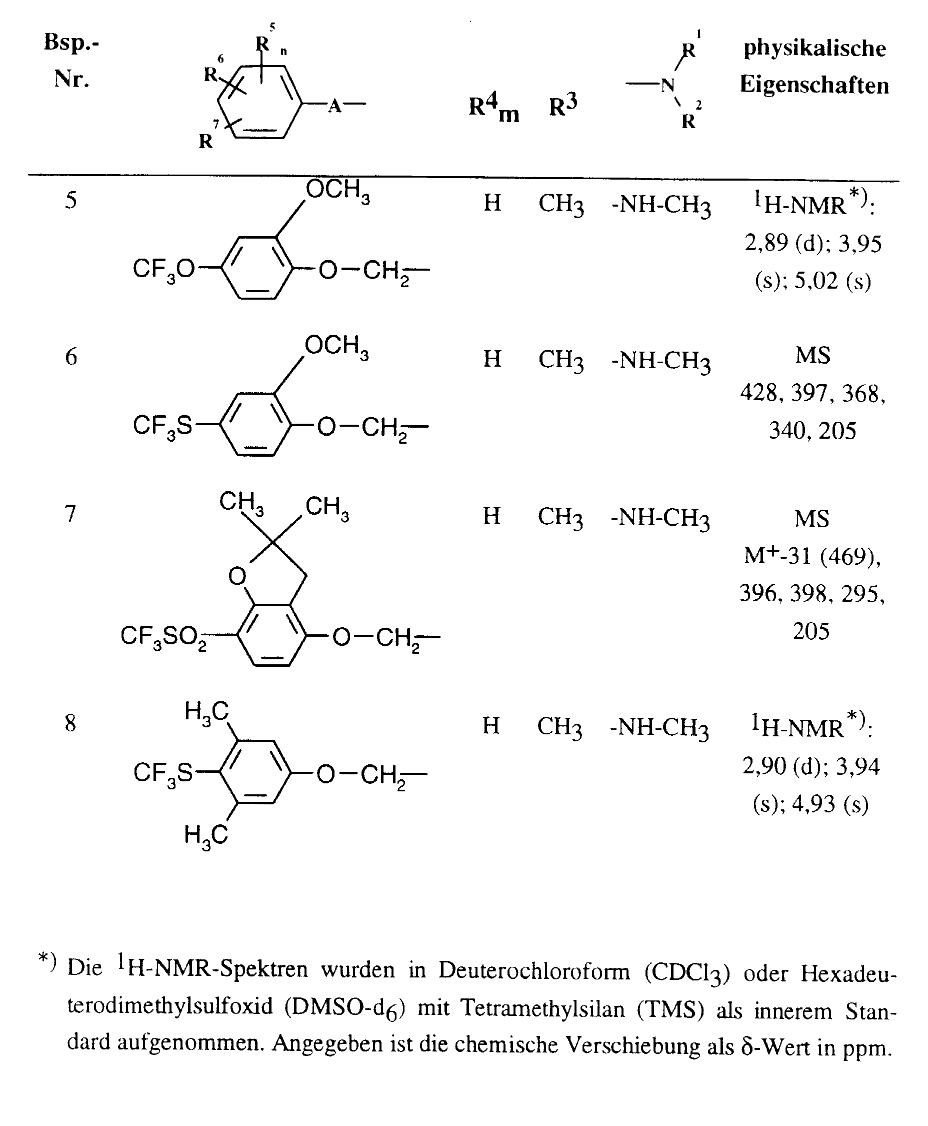

### Anwendungsbeispiele:

In den folgenden Anwendungsbeispielen wurde die nachstehend aufgeführte Verbindungen als Vergleichssubstanz eingesetzt: 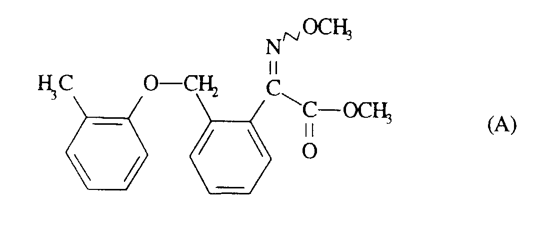 2-Methoximino-2-[2-(2-methyl-phenoxymethyl)-phenyl]-essigsäuremethylester (bekannt aus EP-OS 0 400 417)

### Beispiel A:

### Phytophthora-Test (Tomate) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden dann in einer Inkubationskabine bei 20°C und einer relativen Luftfeuchtigkeit von ca. 100 % aufgestellt.

3 Tage nach der Inokulation erfolgt die Auswertung.

In diesem Test zeigt der im Beispiel 1 aufgeführte erfindungsgemäße Stoff bei einer Wirkstoffkonzentration von 10 ppm in der Spritzflüssigkeit einen Wirkungsgrad von über 80 %, während die Vergleichssubstanz (A) nur einen Wirkungsgrad von 71 % aufweist.

### Beispiel B:

### Plasmopara-Test (Rebe) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Plasmopara viticola inokuliert und verbleiben dann einen Tag in einer Feuchtkammer bei 20°C bis 22°C und 100% relativer Luftfeuchtigkeit. Anschließend werden die Pflanzen 5 Tage im Gewächshaus bei 21°C und 90% Luftfeuchtigkeit aufgestellt. Die Pflanzen werden dann angefeuchtet und einen Tag in eine Feuchtkammer gestellt.

6 Tage nach der Inokulation erfolgt die Auswertung.

In diesem Test zeigt der im Beispiel 1 aufgeführte erfindungsgemäße Stoff bei einer Wirkstoffkonzentration von 2,5 ppm in der Spritzflüssigkeit einen Wirkungsgrad von über 80 %, während die Vergleichssubstanz (A) einen Wirkungsgrad von 40 % aufweist.

### Beispiel C:

### Sclerotinia-Test (Buschbohne) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt zwei kleine mit Sclerotinia sclerotiorum bewachsenen Agarstückchen aufgelegt. Die inokulierten Pflanzen werden in einer abgedunkelten feuchten Kammer bei 20°C aufgestellt.

3 Tage nach der Inokulation wird die Größe der Befallsflecken auf den Blättern ausgewertet.

In diesem Test zeigt der im Beispiel 1 aufgeführte erfindungsgemäße Stoff bei einer Wirkstoffkonzentration von 100 ppm in der Spritzflüssigkeit einen Wirkungsgrad von über 90 %, während die Vergleichssubstanz (A) einen Wirkungsgrad von 74 % aufweist.

### Beispiel D:

### Pseudocercosporella herpotrichoides-Test (Weizen) / protektiv

| | |
|---|---|
| Lösungsmittel | 12,4 Gewichtsteile Dimethylformamid |
| Emulgator | 0,6 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Menge. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

In diesem Test zeigt der im Beispiel 1 aufgeführte erfindungsgemäße Stoff bei einer Wirkstoffkonzentration von 400 g/ha einen Wirkungsgrad von 100 %, während die Vergleichssubstanz (A) keine Wirkung aufweist.

## Patentansprüche

1. 2-Oximino-2-phenyl-acetamide der Formel (I) gemäß Anspruch 1, in denen 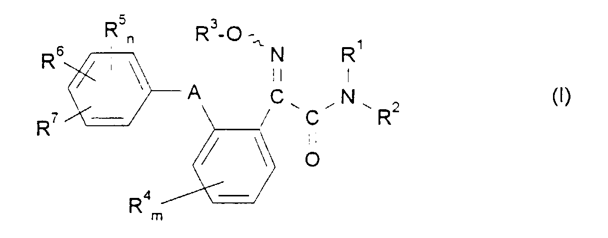
R¹ für Wasserstoff, geradkettiges oder verzweigtes Alkyl mit 1 bis 8 Kohlenstoffatomen oder geradkettiges oder verzweigtes Alkoxy mit 1 bis 8 Kohlenstoffatomen steht,
R² für Wasserstoff, geradkettiges oder verzweigtes Alkyl mit 1 bis 8 Kohlenstoffatomen oder geradkettiges oder verzweigtes Alkoxy mit 1 bis 8 Kohlenstoffatomen steht,
R³ für Wasserstoff, geradkettiges oder verzweigtes Alkyl mit 1 bis 8 Kohlenstoffatomen oder geradkettiges oder verzweigtes Alkoxy mit 1 bis 8 Kohlenstoffatomen steht,
R⁴ für Halogen, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkyl-bzw. Alkoxyteilen steht, außerdem für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
ferner für gegebenenfalls einfach bis dreifach, gleichartig oder verschieden durch Halogen und/oder Alkyl mit 1 bis 4 Kohlenstoffatomen substituiertes 3-bis 7-gliedriges Heterocyclyl mit 2 bis 6 Kohlenstoffatomen und 1 bis 3 gleichen oder verschiedenen Heteroatomen steht,
und weiterhin
für Phenyl, Phenoxy, Benzyl, Benzyloxy, Phenylethyl oder Phenylethyloxy steht, wobei jeder dieser Reste im Phenylteil einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen,
R⁵ für Halogen, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 6 Kohlenstoffatomen in den einzelnen Alkyl-bzw. Alkoxyteilen steht, außerdem für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
ferner für gegebenenfalls einfach bis dreifach, gleichartig oder verschieden durch Halogen und/oder Alkyl mit 1 bis 4 Kohlenstoffatomen substituiertes 3-bis 7-gliedriges Heterocyclyl mit 2 bis 6 Kohlenstoffatomen und 1 bis 3 gleichen oder verschiedenen Heteroatomen steht,
und weiterhin,
für Phenyl, Phenoxy, Benzyl, Benzyloxy, Phenylethyl oder Phenylethyloxy steht, wobei jeder dieser Reste im Phenylteil einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen,
m für die Zahlen 0, 1, 2, 3 oder 4 steht,
n für die Zahlen 1, 2 oder 3 steht,
A für eine Gruppierung der Formel -CH₂-O-, -O-CH₂-, -CH₂-S-oder -S-CH₂- steht,
R⁶ für Wasserstoff, Halogen, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl Alkylsulfonyloxy oder Hydroximinoalkyl mit jeweils 1 bis 6 Kohlenstoffatomen in den einzelnen Alkyl- bzw. Alkoxyteilen steht, außerdem für gegebenenfalls einfach bis dreifach, gleichartig oder verschieden durch Halogen und/oder Alkyl mit 1 bis 4 Kohlenstoffatomen substituiertes Cycloalkyl mit 3 bis 7 Kohlenstoffatomen steht,
ferner für gegebenenfalls einfach bis dreifach, gleichartig oder verschieden durch Halogen und/oder Alkyl mit 1 bis 4 Kohlenstoffatomen substituiertes 3-bis 7-gliedriges Heterocyclyl mit 2 bis 6 Kohlenstoffatomen und 1 bis 3 gleichen oder verschiedenen Heteroatomen steht,
und weiterhin
für Phenyl, Phenoxy, Benzyl, Benzyloxy, Phenylethyl oder Phenylethyloxy steht, wobei jeder dieser Reste im Phenylteil einfach bis fünffach, gleichartig oder verschieden substituiert sein kann durch Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen und oder geradkettiges oder verzweigtes Halogenalkoxy mit 1 bis 4 Kohlenstoffatomen und 1 bis 9 gleichen oder verschiedenen Halogenatomen,
und
R⁷ für jeweils geradkettiges oder verzweigtes Halogenalkoxy oder Halogenalkylthio mit jeweils 1 bis 8 Kohlenstoffatomen und 1 bis 17 gleichen oder verschiedenen Halogenatomen steht oder
R⁶ und R⁷ gemeinsam für einen gegebenenfalls einfach bis sechsfach, gleichartig oder verschieden durch Halogen, geradkettiges oder verzweigtes Halogenalkyl mit 1 bis 6 Kohlenstoffatomen und 1 bis 13 gleichen oder verschiedenen Halogenatomen substituierten zweifach verknüpften Alkandiylrest mit 1 bis 5 Kohlenstoffatomen stehen, der 1 bis 3 Sauerstoff- und/oder Schwefelatome enthält.

2. Verbindungen der Formel (I) gemäß Anspruch 1, in denen
R¹ für Wasserstoff, geradkettiges oder verzweigtes Alkyl mit 1 bis 6 Kohlenstoffatomen oder geradkettiges oder verzweigtes Alkoxy mit 1 bis 6 Kohlenstoffatomen steht,
R² für Wasserstoff, geradkettiges oder verzweigtes Alkyl mit 1 bis 6 Kohlenstoffatomen oder geradkettiges oder verzweigtes Alkoxy mit 1 bis 6 Kohlenstoffatomen steht,
R³ für Wasserstoff, geradkettiges oder verzweigtes Alkyl mit 1 bis 6 Kohlenstoffatomen oder geradkettiges oder verzweigtes Alkoxy mit 1 bis 6 Kohlenstoffatomen steht,
R⁴ für Fluor, Chlor, Brom, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Fluor-, Chlor- und/oder Bromatomen, jeweils geradkettiges oder verzweigtes Halogenalkenyl oder Halogenalkenyloxy mit jeweils 2 bis 4 Kohlenstoffatomen und 1 bis 9 Fluor-, Chlor- und/oder Bromatomen, jeweils geradkettiges oder verzweigtes N-Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 4 Kohlenstoffatomen in den einzelnen Alkyl- bzw. Alkoxyteilen,
außerdem für gegebenenfalls einfach bis dreifach, gleichartig oder verschieden durch Fluor, Chlor, Methyl und/oder Ethyl substituiertes Cycloalkyl mit 3 bis 6 Kohlenstoffatomen,
ferner für gegebenenfalls einfach bis dreifach, gleichartig oder verschieden durch Fluor, Chlor, Methyl und/oder Ethyl substituiertes, 3-bis 7-gliedriges Heterocyclyl mit 2 bis 5 Kohlenstoffatomen und 1 bis 3 gleichen oder verschiedenen Heteroatomen, wie Stickstoff, Sauerstoff und/oder Schwefel,
und weiterhin
für Phenyl, Phenoxy, Benzyl, Benzyloxy, Phenylethyl oder Phenylethyloxy, wobei jeder dieser Reste im Phenylteil einfach bis fünffach, gleichartig oder verschieden substituiert sein kann durch Fluor, Chlor, Brom, geradkettiges oder verzweigtes Alkyl mit 1 bis 3 Kohlenstoffatomen, geradkettiges oder verzweigtes Halogenalkyl mit 1 bis 3 Kohlenstoffatomen und 1 bis 7 Fluor-, Chlor- und/oder Bromatomen, geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 Fluor-, Chlor-und/oder Bromatomen steht,
R⁵ für Fluor, Chlor, Brom, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Fluor-, Chlor-und/oder Bromatomen, jeweils geradkettiges oder verzweigtes Halogenalkenyl oder Halogenalkenyloxy mit jeweils 2 bis 4 Kohlenstoffatomen und 1 bis 9 Fluor-, Chlor-und/oder Bromatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 4 Kohlenstoffatomen in den einzelnen Alkyl-bzw. Alkoxyteilen,
außerdem für gegebenenfalls einfach bis dreifach, gleichartig oder verschieden durch Fluor, Chlor, Methyl und/oder Ethyl substituiertes Cycloalkyl mit 3 bis 6 Kohlenstoffatomen,
ferner für gegebenenfalls einfach bis dreifach, gleichartig oder verschieden durch Fluor, Chlor, Methyl und/oder Ethyl substituiertes, 3- bis 7-gliedriges Heterocyclyl mit 2 bis 5 Kohlenstoffatomen und 1 bis 3 gleichen oder verschiedenen Heteroatomen, wie Stickstoff, Sauerstoff und/oder Schwefel,
und weiterhin
für Phenyl, Phenoxy, Benzyl, Benzyloxy, Phenylethyl oder Phenylethyloxy, wobei jeder dieser Reste im Phenylteil einfach bis fünffach, gleichartig oder verschieden substituiert sein kann durch Fluor, Chlor, Brom, geradkettiges oder verzweigtes Alkyl mit 1 bis 3 Kohlenstoffatomen, geradkettiges oder verzweigtes Halogenalkyl mit 1 bis 3 Kohlenstoffatomen und 1 bis 7 Fluor-, Chlor-und/oder Bromatomen, geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 Fluor-, Chlor-und/oder Bromatomen steht,
m für die Zahlen 0, 1, 2 oder 3 steht, wobei R⁴ für gleiche oder verschiedene Reste stehen kann, wenn m für 2 oder 3 steht,
n für die Zahlen 1, 2 oder 3 steht, wobei R⁵ für gleiche oder verschiedene Reste stehen kann, wenn n für 2 oder 3 steht,
A für eine Gruppierung der Formel -CH₂-O-, -O-CH₂-, -CH₂-S-oder -S-CH₂- steht,
R⁶ für Wasserstoff, Fluor, Chlor, Brom, Cyano, Nitro, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 Fluor-, Chlor-und/oder Bromatomen, jeweils geradkettiges oder verzweigtes Halogenalkenyl oder Halogenalkenyloxy mit jeweils 2 bis 4 Kohlenstoffatomen und 1 bis 9 Fluor-, Chlor- und/oder Bromatomen, jeweils geradkettiges oder verzweigtes N-Alkylamino, Dialkylamino, Alkylcarbonyl, Alkylcarbonyloxy, Alkoxycarbonyl, Alkylsulfonyloxy oder Hydroximinoalkyl mit jeweils 1 bis 4 Kohlenstoffatomen in den einzelnen Alkyl-bzw. Alkoxyteilen,
außerdem für gegebenenfalls einfach bis dreifach, gleichartig oder verschieden durch Fluor, Chlor, Methyl und/oder Ethyl substituiertes Cycloalkyl mit 3 bis 6 Kohlenstoffatomen,
ferner für gegebenenfalls einfach bis dreifach, gleichartig oder verschieden durch Fluor, Chlor, Methyl und/oder Ethyl substituiertes, 3- bis 7-gliedriges Heterocyclyl mit 2 bis 5 Kohlenstoffatomen und 1 bis 3 gleichen oder verschiedenen Heteroatomen, wie Stickstoff, Sauerstoff und/oder Schwefel,
und weiterhin
für Phenyl, Phenoxy, Benzyl, Benzyloxy, Phenylethyl oder Phenylethyloxy, wobei jeder dieser Reste im Phenylteil einfach bis fünffach, gleichartig oder verschieden substituiert sein kann durch Fluor, Chlor, Brom, geradkettiges oder verzweigtes Alkyl mit 1 bis 3 Kohlenstoffatomen, geradkettiges oder verzweigtes Halogenalkyl mit 1 bis 3 Kohlenstoffatomen und 1 bis 7 Fluor-, Chlor-und/oder Bromatomen, geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 Fluor-, Chlor- und/oder Bromatomen steht,
R⁷ für jeweils geradkettiges oder verzweigtes Halogenalkoxy oder Halogenalkylthio mit jeweils 1 bis 4 Kohlenstoffatomen und 1 bis 9 Fluor-, Chlor-und/oder Bromatomen steht,
R⁶ und R⁷ außerdem auch gemeinsam für einen gegebenenfalls einfach bis sechsfach, gleichartig oder verschieden durch Fluor, Chlor, Brom, geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 Fluor-, Chlor- und/oder Bromatomen substituierten zweifach verknüpften Alkandiylrest mit 1 bis 4 Kohlenstoffatomen, der 1 oder 2 Sauerstoff-und/oder Schwefelatome enthält stehen.

3. Verbindungen der Formel (I) gemäß Anspruch 1, in denen
R¹ für Wasserstoff, geradkettiges oder verzweigtes Alkyl mit 1 bis 4 Kohlenstoffatomen oder für geradkettiges oder verzweigtes Alkoxy mit 1 bis 4 Kohlenstoffatomen steht,
R² für Wasserstoff, geradkettiges oder verzweigtes Alkyl mit 1 bis 4 Kohlenstoffatomen oder für geradkettiges oder verzweigtes Alkoxy mit 1 bis 4 Kohlenstoffatomen steht,
R³ für Wasserstoff, geradkettiges oder verzweigtes Alkyl mit 1 bis 4 Kohlenstoffatomen oder für geradkettiges oder verzweigtes Alkoxy mit 1 bis 4 Kohlenstoffatomen steht,
R⁴ für Fluor, Chlor, Brom, Cyano, Nitro, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, Methylsulfinyl, Methylsulfonyl, Allyl, Butenyl, Allyloxy, Butenyloxy, Trifluormethyl, Trifluormethoxy Difluormethoxy, Trifluormethylthio, Difluorchlormethylthio, Trifluormethyl sulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Cyclopropyl, Cyclopentyl, Cyclohexyl, 1-Pyrrolidinyl, 1-Piperidinyl, 1-Perhydroazepinyl, 4-Morpholinyl oder für Phenyl, Phenoxy, Benzyl, Benzyloxy, Phenylethyl oder Phenylethyloxy, wobei jeder der sechs letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy steht,
R⁵ für Fluor, Chlor, Brom, Cyano, Nitro, Formyl, Carbamoyl, Thiocarbamoyl, Methyl, Ethyl, n-oder 1-Propyl, n-, i-, s-oder t-Butyl, Methoxy, Ethoxy, n-oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Cyclopropyl, Cyclopentyl, Cyclohexyl, 1-Pyrrolidinyl, 1-Piperidinyl, 1-Perhydroazepinyl, 4-Morpholinyl oder für Phenyl, Phenoxy, Benzyl, Benzyloxy, Phenylethyl oder Phenylethyloxy, wobei jeder der sechs letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy steht,
m für die Zahlen 0, 1 oder 2 steht, wobei R⁴ für gleiche oder verschiedene Reste stehen kann, wenn m für 2 steht,
n für die Zahlen 1 oder 2 steht, wobei R⁵ für gleiche oder verschiedene Reste stehen kann, wenn n für 2 steht.
A für eine Gruppierung der Formel -CH₂-O-, -O-CH₂-, -CH₂-S-oder -S-CH₂- steht,
R⁶ für Wasserstoff, Fluor, Chlor, Brom, Cyano, Formyl, Formyl, Carbamoyl, Thiocarbamoyl, Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, Methylsulfinyl, Methylsulfonyl, Allyl, Butenyl, Allyloxy, Butenyloxy, Trifluormethyl, Trifluormethoxy, Difluormethoxy, Trifluormethylthio, Difluorchlormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Cyclopropyl, Cyclopentyl, Cyclohexyl, 1-Pyrrolidinyl, 1-Piperidinyl, 1-Perhydroazepinyl, 4-Morpholinyl oder für Phenyl, Phenoxy, Benzyl, Benzyloxy, Phenylethyl oder Phenylethyloxy, wobei jeder der sechs letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy steht,
R⁷ für geradkettiges oder verzweigtes Halogenalkoxy mit 1 bis 3 Kohlenstoffatomen und 1 bis 7 Fluor-, Chlor-und/oder Bromatomen oder für geradkettiges oder verzweigtes Halogenalkylthio mit 1 bis 3 Kohlenstoffatomen und 1 bis 7 Fluor-, Chlor-und/oder Bromatomen steht,
R⁶ und R⁷ außerdem gemeinsam für einen gegebenenfalls einfach bis vierfach, gleich oder verschieden durch Fluor, Chlor, Brom, Methyl, Ethyl und/oder Trifluormethyl substituierten zweifach verknüpften Alkandiylrest mit 1 bis 3 Kohlenstoffatomen, der 1 bis 2 Sauerstoff-oder Schwefelatome enthält, stehen.

4. Verfahren zur Herstellung von 2-Oximino-2-phenyl-acetamiden der Formel 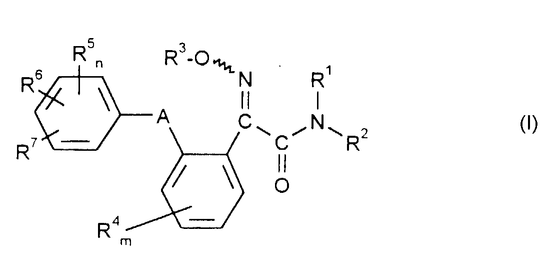 in welcher
R¹, R², R³, R⁴, R⁵, R⁶, R⁷, m, n und A die in Anspruch 1 angegebenen Bedeutungen haben,
dadurch gekennzeichnet, daß man 2-Oximino-2-phenyl-essigsäureester der Formel 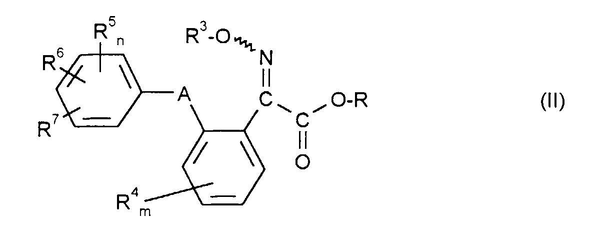 in welcher
R², R⁴, R⁵, R⁶, R⁷, A, m und n die oben angegebenen Bedeutungen haben und
R für Alkyl steht,
mit Ammoniak oder Aminen der Formel 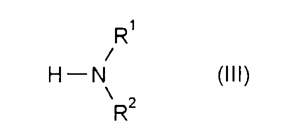 in welcher
R¹ und R² die oben angegebenen Bedeutungen haben,
gegebenenfalls in Gegenwart eines Verdünnungsmittels und gegebenenfalls in Gegenwart eines Säurebindemittels umsetzt.

5. Schädlingsbekämpfungsmittel, gekennzeichnet durch einen Gehalt an mindestens einem 2-Oximino-2-phenyl-acetamid der Formel (I) gemäß Anspruch 1.

6. Verwendung von 2-Oximino-2-phenyl-acetamiden der Formel (I) gemäß Anspruch 1 zur Bekämpfung von Schädlingen.

7. Verfahren zur Bekämpfung von Schädlingen, dadurch gekennzeichnet, daß man 2-Oximino-2-phenyl-acetamide der Formel (I) gemäß Anspruch 1 auf die Schädlinge und/oder deren Lebensraum ausbringt.

8. Verfahren zur Herstellung von Schädlingsbekämpfungsmitteln, dadurch gekennzeichnet, daß man 2-Oximino-2-phenyl-acetamide der Formel (I) gemäß Anspruch 1 mit Streckmitteln und/oder oberflächenaktiven Stoffen vermischt.

## Claims

1. 2-Oximino-2-phenyl-acetamides of the formula (I) according to Claim 1 in which 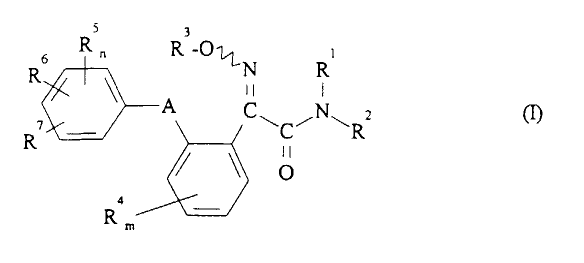
R¹ represents hydrogen, straight-chain or branched alkyl having 1 to 8 carbon atoms or straight-chain or branched alkoxy having 1 to 8 carbon atoms,
R² represents hydrogen, straight-chain or branched alkyl having 1 to 8 carbon atoms or straight-chain or branched alkoxy having 1 to 8 carbon atoms,
R³ represents hydrogen, straight-chain or branched alkyl having 1 to 8 carbon atoms or straight-chain or branched alkoxy having 1 to 8 carbon atoms,
R⁴ represents halogen, cyano, nitro, formyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl or alkenyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched N-alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl or alkoxy moieties,
furthermore represents cycloalkyl having 3 to 7 carbon atoms which is optionally monosubstituted to trisubstituted by identical or different substituents from the series consisting of halogen and/or alkyl having 1 to 4 carbon atoms,
further represents 3- to 7-membered heterocyclyl which has 2 to 6 carbon atoms and 1 to 3 identical or different hetero atoms and which is optionally monosubstituted to trisubstituted by identical or different substituents from the series consisting of halogen and/or alkyl having 1 to 4 carbon atoms,
and moreover
represents phenyl, phenoxy, benzyl, benzyloxy, phenylethyl or phenylethyloxy, it being possible for each of these radicals to be monosubstituted to pentasubstituted in the phenyl moiety by identical or different substituents from the series consisting of halogen, straight-chain or branched alkyl having 1 to 4 carbon atoms, straight-chain or branched halogenoalkyl having 1 to 4 carbon atoms and 1 to 9 identical or different halogen atoms, straight-chain or branched alkoxy having 1 to 4 carbon atoms and/or straight-chain or branched halogenoalkoxy having 1 to 4 carbon atoms and 1 to 9 identical or different halogen atoms,
R⁵ represents halogen, cyano, nitro, formyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl or alkenyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched N-alkylamino, dialkylamino, alkylcarbonyl, alkylcarbonyloxy, alkoxycarbonyl, alkylsulphonyloxy or hydroximinoalkyl, each of which has 1 to 6 carbon atoms in the individual alkyl or alkoxy moieties, furthermore represents cycloalkyl having 3 to 7 carbon atoms which is optionally monosubstituted to trisubstituted by identical or different substituents from the series consisting of halogen and/or alkyl having 1 to 4 carbon atoms,
further represents 3- to 7-membered heterocyclyl which has 2 to 6 carbon atoms and 1 to 3 identical or different hetero atoms and which is optionally monosubstituted to trisubstituted by identical or different substituents from the series consisting of halogen and/or alkyl having 1 to 4 carbon atoms,
and moreover
represents phenyl, phenoxy, benzyl, benzyloxy, phenylethyl or phenylethyloxy, it being possible for each of these radicals to be monosubstituted to pentasubstituted in the phenyl moiety by identical or different substituents from the series consisting of halogen, straight-chain or branched alkyl having 1 to 4 carbon atoms, straight-chain or branched halogenoalkyl having 1 to 4 carbon atoms and 1 to 9 identical or different halogen atoms, straight-chain or branched alkoxy having 1 to 4 carbon atoms, straight-chain or branched halogenoalkoxy having 1 to 4 carbon atoms and 1 to 9 identical or different halogen atoms,
m represents the numbers 0, 1, 2, 3 or 4,
n represents the numbers 1, 2 or 3,
A represents a group of the formula -CH₂-O-, -O-CH₂-, -CH₂-S- or -S-CH₂-,
R⁶ represents hydrogen, halogen, cyano, nitro, formyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl or alkenyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched N-alkylamino, dialkylamino, alkylcarbonyl, alkylcarbonyloxy, alkoxycarbonyl, alkylsulphonyloxy or hydroximinoalkyl, each of which has 1 to 6 carbon atoms in the individual alkyl or alkoxy moieties, furthermore represents cycloalkyl having 3 to 7 carbon atoms which is optionally monosubstituted to trisubstituted by identical or different substituents from the series consisting of halogen and/or alkyl having 1 to 4 carbon atoms,
further represents 3- to 7-membered heterocyclyl which has 2 to 6 carbon atoms and 1 to 3 identical or different hetero atoms and which is optionally monosubstituted to trisubstituted by identical or different substituents from the series consisting of halogen and/or alkyl having 1 to 4 carbon atoms,
and moreover
represents phenyl, phenoxy, benzyl, benzyloxy, phenylethyl or phenylethyloxy, it being possible for each of these radicals to be monosubstituted to pentasubstituted in the phenyl moiety by identical or different substituents from the series consisting of halogen, straight-chain or branched alkyl having 1 to 4 carbon atoms, straight-chain or branched halogenoalkyl having 1 to 4 carbon atoms and 1 to 9 identical or different halogen atoms, straight-chain or branched alkoxy having 1 to 4 carbon atoms and/or straight-chain or branched halogenoalkoxy having 1 to 4 carbon atoms and 1 to 9 identical or different halogen atoms,
and
R⁷ represents in each case straight-chain or branched halogenoalkoxy or halogenoalkylthio, each of which has 1 to 8 carbon atoms and 1 to 17 identical or different halogen atoms or
R⁶ and R⁷ together represent a bivalent alkanediyl radical having 1 to 5 carbon atoms which contains 1 to 3 oxygen and/or sulphur atoms and which is optionally monosubstituted to hexasubstituted by identical or different substituents from the series consisting of halogen, straight-chain or branched halogenoalkyl having 1 to 6 carbon atoms and 1 to 13 identical or different halogen atoms.

2. Compounds of the formula (I) according to Claim 1 in which
R¹ represents hydrogen, straight-chain or branched alkyl having 1 to 6 carbon atoms or straight-chain or branched alkoxy having 1 to 6 carbon atoms,
R² represents hydrogen, straight-chain or branched alkyl having 1 to 6 carbon atoms or straight-chain or branched alkoxy having 1 to 6 carbon atoms,
R³ represents hydrogen, straight-chain or branched alkyl having 1 to 6 carbon atoms or straight-chain or branched alkoxy having 1 to 6 carbon atoms,
R⁴ represents fluorine, chlorine, bromine, cyano, nitro, formyl, carbamoyl, thiocarbamoyl, in each case straight-chain or branched alkyl, alkoxy, alkylthio, alkylsulphinyl or alkylsulphonyl, each of which has 1 to 4 carbon atoms, in each case straight-chain or branched alkenyl or alkenyloxy, each of which has 2 to 4 carbon atoms, in each case straight-chain or branched halogenoalkyl, halogenoalkoxy, halogenoalkylthio, halogenoalkylsulphinyl or halogenoalkylsulphonyl, each of which has 1 to 4 carbon atoms and 1 to 9 fluorine, chlorine and/or bromine atoms, in each case straight-chain or branched halogenoalkenyl or halogenoalkenyloxy, each of which has 2 to 4 carbon atoms and 1 to 9 fluorine, chlorine and/or bromine atoms, in each case straight-chain or branched N-alkylamino, dialkylamino, alkylcarbonyl, alkylcarbonyloxy, alkoxycarbonyl, alkylsulphonyloxy, hydroximinoalkyl or alkoximinoalkyl, each of which has 1 to 4 carbon atoms in the individual alkyl or alkoxy moieties,
furthermore represents cycloalkyl having 3 to 6 carbon atoms which is optionally monosubstituted to trisubstituted by identical or different substituents from the series consisting of fluorine, chlorine, methyl and/or ethyl,
further represents 3- to 7-membered heterocyclyl which has 2 to 5 carbon atoms and 1 to 3 identical or different hetero atoms, such as nitrogen, oxygen and/or sulphur, and which is optionally monosubstituted to trisubstituted by identical or different substituents from the series consisting of fluorine, chlorine, methyl and/or ethyl,
and moreover
represents phenyl, phenoxy, benzyl, benzyloxy, phenylethyl or phenylethyloxy, it being possible for each of these radicals to be monosubstituted to pentasubstituted in the phenyl moiety by identical or different substituents from the series consisting of fluorine, chlorine, bromine, straight-chain or branched alkyl having 1 to 3 carbon atoms, straight-chain or branched halogenoalkyl having 1 to 3 carbon atoms and 1 to 7 fluorine, chlorine and/or bromine atoms, straight-chain or branched alkoxy having 1 to 3 carbon atoms and/or straight-chain or branched halogenoalkoxy having 1 to 3 carbon atoms and 1 to 7 fluorine, chlorine and/or bromine atoms,
R⁵ represents fluorine, chlorine, bromine, cyano, nitro, formyl, carbamoyl, thiocarbamoyl, in each case straight-chain or branched alkyl, alkoxy, alkylthio, alkylsulphinyl or alkylsulphonyl, each of which has 1 to 4 carbon atoms, in each case straight-chain or branched alkenyl or alkenyloxy, each of which has 2 to 4 carbon atoms, in each case straight-chain or branched halogenoalkyl, halogenoalkoxy, halogenoalkylthio, halogenoalkylsulphinyl or halogenoalkylsulphonyl, each of which has 1 to 4 carbon atoms and 1 to 9 fluorine, chlorine and/or bromine atoms, in each case straight-chain or branched halogenoalkenyl or halogenoalkenyloxy, each of which has 2 to 4 carbon atoms and 1 to 9 fluorine, chlorine and/or bromine atoms, in each case straight-chain or branched N-alkylamino, dialkylamino, alkylcarbonyl, alkylcarbonyloxy, alkoxycarbonyl, alkylsulphonyloxy or hydroximinoalkyl, each of which has 1 to 4 carbon atoms in the individual alkyl or alkoxy moieties,
furthermore represents cycloalkyl having 3 to 6 carbon atoms which is optionally monosubstituted to trisubstituted by identical or different substituents from the series consisting of fluorine, chlorine, methyl and/or ethyl,
further represents 3- to 7-membered heterocyclyl which has 2 to 5 carbon atoms and 1 to 3 identical or different hetero atoms, such as nitrogen, oxygen and/or sulphur, and which is optionally monosubstituted to trisubstituted by identical or different substituents from the series consisting of fluorine, chlorine, methyl and/or ethyl,
and moreover
represents phenyl, phenoxy, benzyl, benzyloxy, phenylethyl or phenylethyloxy, it being possible for each of these radicals to be monosubstituted to pentasubstituted in the phenyl moiety by identical or different substituents from the series consisting of fluorine, chlorine, bromine, straight-chain or branched alkyl having 1 to 3 carbon atoms, straight-chain or branched halogenoalkyl having 1 to 3 carbon atoms and 1 to 7 fluorine, chlorine and/or bromine atoms, straight-chain or branched alkoxy having 1 to 3 carbon atoms and/or straight-chain or branched halogenoalkoxy having 1 to 3 carbon atoms and 1 to 7 fluorine, chlorine and/or bromine atoms,
m represents the numbers 0, 1, 2 or 3, it being possible for R⁴ to represent identical or different radicals if m represents 2 or 3,
n represents the numbers 1, 2 or 3, it being possible for R⁵ to represent identical or different radicals if n represents 2 or 3,
A represents a group of the formula -CH₂-O-, -O-CH₂-, -CH₂-S- or -S-CH₂-,
R⁶ represents hydrogen, fluorine, chlorine, bromine, cyano, nitro, formyl, carbamoyl, thiocarbamoyl, in each case straight-chain or branched alkyl, alkoxy, alkylthio, alkylsulphinyl or alkylsulphonyl, each of which has 1 to 4 carbon atoms, in each case straight-chain or branched alkenyl or alkenyloxy, each of which has 2 to 4 carbon atoms, in each case straight-chain or branched halogenoalkyl, halogenoalkoxy, halogenoalkylthio, halogenoalkylsulphinyl or halogenoalkylsulphonyl, each of which has 1 to 4 carbon atoms and 1 to 9 fluorine, chlorine and/or bromine atoms, in each case straight-chain or branched halogenoalkenyl or halogenoalkenyloxy, each of which has 2 to 4 carbon atoms and 1 to 9 fluorine, chlorine and/or bromine atoms, in each case straight-chain or branched N-alkylamino, dialkylamino, alkylcarbonyl, alkylcarbonyloxy, alkoxycarbonyl, alkylsulphonyloxy or hydroximinoalkyl, each of which has 1 to 4 carbon atoms in the individual alkyl or alkoxy moieties,
furthermore represents cycloalkyl having 3 to 6 carbon atoms which is optionally monosubstituted to trisubstituted by identical or different substituents from the series consisting of fluorine, chlorine, methyl and/or ethyl,
further represents 3- to 7-membered heterocyclyl which has 2 to 5 carbon atoms and 1 to 3 identical or different hetero atoms, such as nitrogen, oxygen and/or sulphur, and which is optionally monosubstituted to trisubstituted by identical or different substituents from the series consisting of fluorine, chlorine, methyl and/or ethyl,
and moreover
represents phenyl, phenoxy, benzyl, benzyloxy, phenylethyl or phenylethyloxy, it being possible for each of these radicals to be monosubstituted to pentasubstituted in the phenyl moiety by identical or different substituents from the series consisting of fluorine, chlorine, bromine, straight-chain or branched alkyl having 1 to 3 carbon atoms, straight-chain or branched halogenoalkyl having 1 to 3 carbon atoms and 1 to 7 fluorine, chlorine and/or bromine atoms, straight-chain or branched alkoxy having 1 to 3 carbon atoms and/or straight-chain or branched halogenoalkoxy having 1 to 3 carbon atoms and 1 to 7 fluorine, chlorine and/or bromine atoms,
R⁷ represents in each case straight-chain or branched halogenoalkoxy or halogenoalkylthio, each of which has 1 to 4 carbon atoms and 1 to 9 fluorine, chlorine and/or bromine atoms,
R⁶ and R⁷ furthermore together also represent a bivalent alkanediyl radical having 1 to 4 carbon atoms which contains 1 or 2 oxygen and/or sulphur atoms and which is optionally monosubstituted to hexasubstituted by identical or different substituents from the series consisting of fluorine, chlorine, bromine, straight-chain or branched alkyl having 1 to 4 carbon atoms and/or straight-chain or branched halogenoalkyl having 1 to 4 carbon atoms and 1 to 9 fluorine, chlorine and/or bromine atoms.

3. Compounds of the formula (I) according to Claim 1 in which
R¹ represents hydrogen, straight-chain or branched alkyl having 1 to 4 carbon atoms or straight-chain or branched alkoxy having 1 to 4 carbon atoms,
R² represents hydrogen, straight-chain or branched alkyl having 1 to 4 carbon atoms or straight-chain or branched alkoxy having 1 to 4 carbon atoms,
R³ represents hydrogen, straight-chain or branched alkyl having 1 to 4 carbon atoms or straight-chain or branched alkoxy having 1 to 4 carbon atoms,
R⁴ represents fluorine, chlorine, bromine, cyano, nitro, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, difluoromethoxy, trifluoromethylthio, difluorochloromethylthio, trifluoromethylsulphinyl, trifluoromethylsulphonyl, dimethylamino, diethylamino, acetyl, acetoxy, methylsulphonyloxy, ethylsulphonyloxy, methoxycarbonyl, ethoxycarbonyl, hydroximinomethyl, hydroximinoethyl, methoximinomethyl, methoximinoethyl, ethoximinomethyl, ethoximinoethyl, cyclopropyl, cyclopentyl, cyclohexyl, 1-pyrrolidinyl, 1-piperidinyl, 1-perhydroazepinyl, 4-morpholinyl, or represents phenyl, phenoxy, benzyl, benzyloxy, phenylethyl or phenylethyloxy, it being possible for each of the six last-mentioned radicals to be mono-substituted to trisubstituted in the phenyl moiety by identical or different substituents from the series consisting of fluorine, chlorine, bromine, methyl, ethyl, methoxy, ethoxy, trifluoromethyl and/or trifluoromethoxy,
R⁵ represents fluorine, chlorine, bromine, cyano, nitro, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, difluoromethoxy, trifluoromethylthio, difluorochloromethylthio, trifluoromethylsulphinyl, trifluoromethylsulphonyl, dimethylamino, diethylamino, acetyl, acetoxy, methylsulphonyloxy, ethylsulphonyloxy, methoxycarbonyl, ethoxycarbonyl, hydroximinomethyl, hydroximinoethyl, cyclopropyl, cyclopentyl, cyclohexyl, 1-pyrrolidinyl, 1-piperidinyl, 1-perhydroazepinyl, 4-morpholinyl, or represents phenyl, phenoxy, benzyl, benzyloxy, phenylethyl or phenylethyloxy, it being possible for each of the six last-mentioned radicals to be mono-substituted to trisubstituted in the phenyl moiety by identical or different substituents from the series consisting of fluorine, chlorine, bromine, methyl, ethyl, methoxy, ethoxy, trifluoromethyl and/or trifluoromethoxy,
m represents the numbers 0, 1 or 2, it being possible for R⁴ to represent identical or different radicals if m represents 2,
n represents the numbers 1 or 2, it being possible for R⁵ to represent identical or different radicals if n represents 2,
A represents a group of the formula -CH₂-O-, -O-CH₂-, -CH₂-S- or -S-CH₂-,
R⁶ represents hydrogen, fluorine, chlorine, bromine, cyano, nitro, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, difluoromethoxy, trifluoromethylthio, difluorochloromethylthio, trifluoromethylsulphinyl, trifluoromethylsulphonyl, dimethylamino, diethylamino, acetyl, acetoxy, methylsulphonyloxy, ethylsulphonyloxy, methoxycarbonyl, ethoxycarbonyl, hydroximinomethyl, hydroximinoethyl, cyclopropyl, cyclopentyl, cyclohexyl, 1-pyrrolidinyl, 1-piperidinyl, 1-perhydroazepinyl, 4-morpholinyl, or represents phenyl, phenoxy, benzyl, benzyloxy, phenylethyl or phenylethyloxy, it being possible for each of the six last-mentioned radicals to be monosubstituted to trisubstituted in the phenyl moiety by identical or different substituents from the series consisting of fluorine, chlorine, bromine, methyl, ethyl, methoxy, ethoxy, trifluoromethyl and/or trifluoromethoxy,
R⁷ represents straight-chain or branched halogenoalkoxy having 1 to 3 carbon atoms and 1 to 7 fluorine, chlorine and/or bromine atoms, or represents straight-chain or branched halogenoalkylthio having 1 to 3 carbon atoms and 1 to 7 fluorine, chlorine and/or bromine atoms,
R⁶ and R⁷ furthermore together represent a bivalent alkanediyl radical having 1 to 3 carbon atoms which contains 1 or 2 oxygen and/or sulphur atoms and which is optionally monosubstituted to tetrasubstituted by identical or different substituents from the series consisting of fluorine, chlorine, bromine, methyl, ethyl and/or trifluoromethyl,

4. Process for the preparation of 2-oximino-2-phenylacetamides of the formula 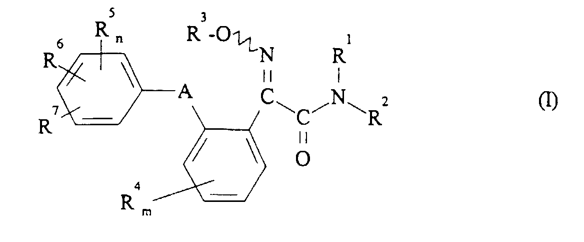 in which
R¹, R², R³, R⁴, R⁵, R⁶, R⁷, m, n and A have the meanings mentioned in Claim 1,
characterized in that 2-oximino-2-phenyl-acetates of the formula 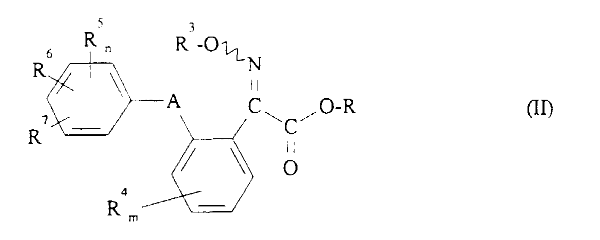 in which
R³, R⁴, R⁵, R⁶, R⁷, A m and n have the abovementioned meanings and
R represents alkyl
are reacted with ammonia or amines of the formula 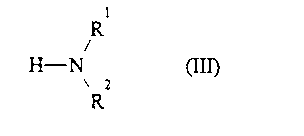 in which
R¹ and R² have the abovementioned meanings,
if appropriate in the presence of a diluent and if appropriate in the presence of an acid-binding agent.

5. Pesticides, characterized in that they contain at least one 2-oximino-2-phenyl-acetamide of the formula (I) according to Claim 1.

6. Use of 2-oximino-2-phenyl-acetamides of the formula (I) according to Claim 1 for combating pests.

7. Method of combating pests, characterized in that 2-oximino-2-phenyl-acetamides of the formula (I) according to Claim 1 are applied to the pests and/or their environment.

8. Process for the preparation of pesticides, characterized in that 2-oximino-2-phenyl-acetamides of the formula (I) according to Claim 1 are mixed with extenders and/or surface-active substances.

## Revendications

1. 2-oximino-2-phénylacétamides de formule (I) 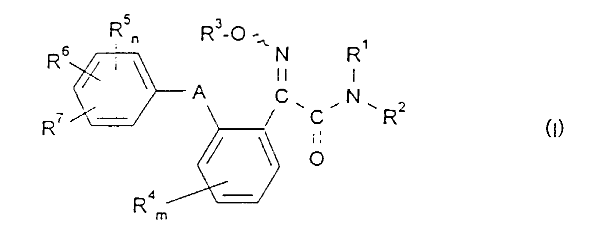 dans laquelle
R¹ représente de l'hydrogène, un groupe alkyle linéaire ou ramifié ayant 1 à 8 atomes de carbone ou un groupe alkoxy linéaire ou ramifié ayant 1 à 8 atomes de carbone,
R² est de l'hydrogène, un groupe alkyle linéaire ou ramifié ayant 1 à 8 atomes de carbone ou un groupe alkoxy linéaire ou ramifié ayant 1 à 8 atomes de carbone,
R³ représente de l'hydrogène, un groupe alkyle linéaire ou ramifié ayant 1 à 8 atomes de carbone ou un groupe alkoxy linéaire ou ramifié ayant 1 à 8 atomes de carbone,
R⁴ représente un halogène, un groupe cyano, nitro, formyle, carbamoyle, thiocarbamoyle, un groupe alkyle, alkoxy, alkylthio, alkylsulfinyle ou alkylsulfonyle étant chacun linéaire ou ramifié et ayant chacun 1 à 6 atomes de carbone, un groupe alcényle ou alcényloxy étant chacun linéaire ou ramifié et ayant chacun 2 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe halogénalcényle ou halogénalcényloxy étant chacun linéaire ou ramifié et ayant chacun 2 à 6 atomes de carbone et 1 à 13 atomes d'halogènes identiques ou différents, un groupe N-alkylamino, dialkylamino, alkylcarbonyle, alkylcarbonyloxy, alkoxycarbonyle, alkylsulfonyloxy, hydroximinoalkyle ou alkoximinoalkyle étant chacun linéaire ou ramifié et ayant chacun 1 à 6 atomes de carbone dans les parties alkyle ou alkoxy individuelles, en outre un groupe cycloalkyle de 3 à 7 atomes de carbone portant, le cas échéant, un à trois substituants, identiques ou différents, halogéno et/ou alkyle ayant 1 à 4 atomes de carbone,
en outre, un groupe hétérocyclyle de 3 à 7 chaînons ayant 1 à 3 hétéroatomes identiques ou différents et portant, le cas échéant, un à trois substituants, identiques ou différents, halogéno et/ou alkyle ayant 1 à 4 atomes de carbone,
et en outre
un reste phényle, phénoxy, benzyle, benzyloxy, phényléthyle ou phényléthyloxy, chacun de ces restes pouvant être substitué une à cinq fois identiques ou différentes dans la partie phényle par un halogène, un groupe alkyle linéaire ou ramifié ayant 1 à 4 atomes de carbone, un groupe halogénalkyle linéaire ou ramifié ayant 1 à 4 atomes de carbone et 1 à 9 atomes d'halogènes identiques ou différents, un groupe alkoxy linéaire ou ramifié ayant 1 à 4 atomes de carbone et/ou un groupe halogénalkoxy linéaire ou ranifié ayant 1 à 4 atomes de carbone et 1 à 9 atomes d'halogènes identiques ou différents,
R⁵ représente un halogène, un groupe cyano, nitro, formyle, carbamoyle, thiocarbamoyle, un groupe alkyle, alkoxy, alkylthio, alkylsulfinyle ou alkylsulfonyle étant chacun linéaire ou ramifié et ayant chacun 1 à 6 atomes de carbone, un groupe alcényle ou alcényloxy étant chacun linéaire ou ramifié et ayant chacun 2 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe halogénalcényle ou halogénalcényloxy étant chacun linéaire ou ramifié et ayant chacun 2 à 6 atomes de carbone et 1 à 13 atomes d'halogènes identiques ou différents, un groupe N-alkylamino, dialkylamino, alkylcarbonyle, alkylcarbonyloxy, alkoxycarbonyle, alkylsulfonyloxy ou hydroximinoalkyle étant chacun linéaire ou ramifié et ayant chacun 1 à 6 atomes de carbone dans les parties alkyle ou alkoxy individuelles, en outre un groupe cycloalkyle de 3 à 7 atomes de carbone éventuellement substitué une à trois fois identiques ou différentes par un halogène et/ou un radical alkyle ayant 1 à 4 atomes de carbone,
en outre un groupe hétérocyclyle de 3 à 7 chaînons ayant 2 à 6 atomes de carbone et 1 à 3 hétéroatomes identiques ou différents, éventuellement substitué une à trois fois identiques ou différentes par un halogène et/ou un radical alkyle ayant 1 à 4 atomes de carbone,
et en outre
un reste phényle, phénoxy, benzyle, benzyloxy, phényléthyle ou phényléthyloxy, chacun de ces restes pouvant être substitué dans la partie phényle une à cinq fois identiques ou différentes par un halogène, un radical alkyle linéaire ou ramifié ayant 1 à 4 atomes de carbone, un radical halogénalkyle linéaire ou ramifié ayant 1 à 4 atomes de carbone et 1 à 9 atomes d'halogènes identiques ou différents, un radical alkoxy linéaire ou ramifié ayant 1 à 4 atomes de carbone, un radical halogénalkoxy linéaire ou ramifié ayant 1 à 4 atomes de carbone et 1 à 9 atomes d'halogènes identiques ou différents,
m représente les nombres 0, 1, 2, 3 ou 4,
n représente les nombres 1, 2 ou 3,
A représente un groupement de formule -CH₂-O-, -O-CH₂-, -CH₂-S- ou -S-CH₂-,
R⁶ est de l'hydrogène, un halogène, un groupe cyano, nitro, formyle, carbamoyle, thiocarbamoyle, un groupe alkyle, alkoxy, alkylthio, alkylsulfinyle ou alkylsulfonyle étant chacun linéaire ou ramifié et ayant chacun 1 à 6 atomes de carbone, un groupe alcényle ou alcényloxy étant chacun linéaire ou ramifié et ayant chacun 2 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe halogénalcényle ou halogénalcényloxy étant chacun linéaire ou ramifié et ayant chacun 2 à 6 atomes de carbone et 1 à 13 atomes d'halogènes identiques ou différents, un groupe N-alkylamino, dialkylamino, alkylcarbonyle, alkylcarbonyloxy, alkoxycarbonyle, alkylsulfonyloxy ou hydroximinoalkyle étant chacun linéaire ou ramifié et ayant chacun 1 à 6 atomes de carbone dans les parties alkyle et alkoxy individuelles, en outre un groupe cycloalkyle de 3 à 7 atomes de carbone éventuellement substitué une à trois fois identiques ou différentes par un halogène et/ou un radical alkyle ayant 1 à 4 atomes de carbone,
en outre un groupe hétérocyclyle de 3 à 7 chaînons ayant 2 à 6 atomes de carbone et 1 à 3 hétéroatomes identiques ou différents, éventuellement substitué une à trois fois identiques ou différentes par un halogène et/ou un radical alkyle ayant 1 à 4 atomes de carbone,
et en outre
un reste phényle, phénoxy, benzyle, benzyloxy, phényléthyle ou phényléthyloxy, chacun de ces restes pouvant être substitué dans la partie phényle une à cinq fois identiques ou différentes par un halogène, un radical alkyle linéaire ou ramifié ayant 1 à 4 atomes de carbone, un radical halogénalkyle linéaire ou ramifié ayant 1 à 4 atomes de carbone et 1 à 9 atomes d'halogènes identiques ou différents, un radical alkoxy linéaire ou ramifié ayant 1 à 4 atomes de carbone et/ou halogénalkoxy linéaire ou ramifié ayant 1 à 4 atomes de carbone et 1 à 9 atomes d'halogènes identiques ou différents,
et
R⁷ représente un groupe halogénalkoxy ou halogénalkylthio étant chacun linéaire ou ramifié et ayant chacun 1 à 8 atomes de carbone et 1 à 17 atomes d'halogènes identiques ou différentes,
ou
R⁶ et R⁷ forment ensemble un reste alcanediyle à deux liaisons ayant 1 à 5 atomes de carbone et contenant 1 à 3 atomes d'oxygène et/ou de soufre, éventuellement substitué une à six fois identiques ou différentes par un halogène, un radical halogénalkyle linéaire ou ramifié ayant 1 à 6 atomes de carbone et 1 à 13 atomes d'halogènes identiques ou différents.

2. Composés de formule (I) suivant la revendication 1, dans lesquels
R¹ représente de l'hydrogène, un groupe alkyle linéaire ou ramifié ayant 1 à 6 atomes de carbone ou un groupe alkoxy linéaire ou ramifié ayant 1 à 6 atomes de carbone,
R² représente de l'hydrogène, un groupe alkyle linéaire ou ramifié ayant 1 à 6 atomes de carbone ou un groupe alkoxy linéaire ou ramifié ayant 1 à 6 atomes de carbone,
R³ est de l'hydrogène, un groupe alkyle linéaire ou ramifié ayant 1 à 6 atomes de carbone ou un groupe alkoxy linéaire ou ramifié ayant 1 à 6 atomes de carbone,
R⁴ représente du fluor, du chlore, du brome, un groupe cyano, nitro, formyle, carbamoyle, thiocarbamoyle, un groupe alkyle, alkoxy, alkylthio, alkylsulfinyle ou alkylsulfonyle étant chacun linéaire ou ramifié et ayant chacun 1 à 4 atomes de carbone, un groupe alcényle ou alcényloxy étant chacun linéaire ou ramifié et ayant chacun 2 à 4 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 4 atomes de carbone et 1 à 9 atomes de fluor, de chlore et/ou de brome, un groupe halogénalcényle ou halogénalcényloxy étant chacun linéaire ou ramifié et ayant chacun 2 à 4 atomes de carbone et 1 à 9 atomes de fluor, de chlore et/ou de brome, un groupe N-alkylamino, dialkylamino, alkylcarbonyle, alkylcarbonyloxy, alkoxycarbonyle, alkylsulfonyloxy, hydroximinoalkyle ou alkoximinoalkyle étant chacun linéaire ou ramifié et ayant chacun 1 à 4 atomes de carbone dans les parties alkyle et alkoxy individuelles, un groupe cycloalkyle de 3 à 6 atomes de carbone éventuellement substitué une à trois fois identiques ou différentes par du fluor, du chlore, un radical méthyle et/ou éthyle,
en outre un groupe hétérocyclyle de 3 à 7 chaînons ayant 2 à 5 atomes de carbone et 1 à 3 hétéroatomes identiques ou différents tels qu'azote, oxygène et/ou soufre, éventuellement substitué une à trois fois identiques ou différentes par du fluor, du chlore, un radical méthyle et/ou éthyle,
et en outre
un reste phényle, phénoxy, benzyle, benzyloxy, phényléthyle ou phényléthyloxy, chacun de ces restes pouvant être substitué dans la partie phényle une à cinq fois identiques ou différentes par du fluor, du chlore, du brome, un radical alkyle linéaire ou ramifié ayant 1 à 3 atomes de carbone, un radical halogénalkyle linéaire ou ramifié ayant 1 à 3 atomes de carbone et 1 à 7 atomes de fluor, de chlore et/ou de brome, un radical alkoxy linéaire ou ranifié ayant 1 à 3 atomes de carbone et/ou un radical halogénalkoxy linéaire ou ramifié ayant 1 à 3 atomes de carbone et 1 à 7 atomes de fluor, de chlore et/ou de brome,
R⁵ représente du fluor, du chlore, du brome, un groupe cyano, nitro, formyle, carbamoyle, thiocarbamoyle, un groupe alkyle, alkoxy, alkylthio, alkylsulfinyle ou alkylsulfonyle étant chacun linéaire ou ramifié et ayant chacun 1 à 4 atomes de carbone, un groupe alcényle ou alcényloxy étant chacun linéaire ou ramifié et ayant chacun 2 à 4 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 4 atomes de carbone et 1 à 9 atomes de fluor, de chlore et/ou de brome, un groupe halogénalcényle ou halogénalcényloxy étant chacun linéaire ou ramifié et ayant chacun 2 à 4 atomes de carbone et 1 à 9 atomes de fluor, de chlore et/ou de brome, un groupe N-alkylamino, dialkylamino, alkylcarbonyle, alkylcarbonyloxy, alkoxycarbonyle, alkylsulfonyloxy ou hydroximinoalkyle étant chacun linéaire ou ramifié et ayant chacun 1 à 4 atomes de carbone dans les parties alkyle et alkoxy individuelles,
en outre un groupe cycloalkyle de 3 à 6 atomes de carbone éventuellement substitué une à trois fois identiques ou différentes par du fluor, du chlore, un radical méthyle et/ou éthyle,
en outre un groupe hétérocyclyle de 3 à 7 chaînons ayant 2 à 5 atomes de carbone et 1 à 3 hétéroatomes identiques ou différents tels qu'azote, oxygène et/ou soufre, éventuellement substitué une à trois fois identiques ou différentes par du fluor, du chlore, un radical méthyle et/ou éthyle,
et en outre
un reste phényle, phénoxy, benzyle, benzyloxy, phényléthyle ou phényléthyloxy, chacun de ces restes pouvant être substitué dans la partie phényle une à cinq fois identiques ou différentes par du fluor, du chlore, du brome, un radical alkyle linéaire ou ramifié ayant 1 à 3 atomes de carbone, un radical halogénalkyle linéaire ou ramifié ayant 1 à 3 atomes de carbone et 1 à 7 atomes de fluor, de chlore et/ou de brome, un radical alkoxy linéaire ou ramifié ayant 1 à 3 atomes de carbone et/ou un radical halogénalkoxy linéaire ou ramifié ayant 1 à 3 atomes de carbone et 1 à 7 atomes de fluor, de chlore et/ou de brome,
m représente les nombres 0, 1, 2 ou 3, R⁴ pouvant désigner des restes identiques ou différents lorsque m la valeur 2 ou 3,
n représente les nombres 1, 2 ou 3, R⁵ pouvant représenter des restes identiques ou différents lorsque n a la valeur 2 ou 3,
A est un groupement de formule -CH₂-O-, -O-CH₂-. -CH₂-S- ou -S-CH₂-,
R⁶ représente de l'hydrogène, du fluor, du chlore, du brome, un groupe cyano, nitro, formyle, carbamoyle, thiocarbamoyle, un groupe alkyle, alkoxy, alkylthio, alkylsulfinyle ou alkylsulfonyle étant chacun linéaire ou ramifié et ayant chacun 1 à 4 atomes de carbone, un groupe alcényle ou alcényloxy étant chacun linéaire ou ranifié et ayant chacun 2 à 4 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 4 atomes de carbone et 1 à 9 atomes de fluor, de chlore et/ou de brome, un groupe halogénalcényle ou halogénalcényloxy étant chacun linéaire ou ramifié et ayant chacun 2 à 4 atomes de carbone et 1 à 9 atomes de fluor, de chlore et/ou de brome, un groupe N-alkylamino, dialkylamino, alkylcarbonyle, alkylcarbonyloxy, alkoxycarbonyle, alkylsulfonyloxy ou hydroximinoalkyle étant chacun linéaire ou ramifié et ayant chacun 1 à 4 atomes de carbone dans les parties alkyle et alkoxy individuelles, en outre un groupe cycloalkyle de 3 à 6 atomes de carbone éventuellement substitué une à trois fois identiques ou différentes par du fluor, du chlore, un radical méthyle et/ou éthyle,
en outre un groupe hétérocyclyle de 3 à 7 chaînons ayant 2 à 5 atomes de carbone et 1 à 3 hétéroatomes identiques ou différents tels qu'azote, oxygène et/ou soufre, éventuellement substitué une à trois fois identiques ou différentes par du fluor, du chlore, un radical méthyle et/ou éthyle,
et en outre
un reste phényle, phénoxy, benzyle, benzyloxy, phényléthyle ou phényléthyloxy, chacun de ces restes pouvant être substitué dans la partie phényle une à cinq fois identiques ou différentes par du fluor, du chlore, du brome, un radical alkyle linéaire ou ramifié ayant 1 à 3 atomes de carbone, un radical halogénalkyle linéaire ou ramifié ayant 1 à 3 atomes de carbone et 1 à 7 atomes de fluor, de chlore et/ou de brome, un radical alkoxy linéaire ou ramifié ayant 1 à 3 atomes de carbone et/ou un radical halogénalkoxy linéaire ou ramifié ayant 1 à 3 atomes de carbone et 1 à 7 atomes de fluor, de chlore et/ou de brome,
R⁷ représente un groupe halogénalkoxy ou halogénalkylthio étant chacun linéaire ou ramifié et ayant chacun 1 à 4 atomes de carbone et 1 à 9 atomes de fluor, de chlore et/ou de brome, en outre
R⁶ et R⁷ forment aussi ensemble un reste alcanediyle à deux liaisons ayant 1 à 4 atomes de carbone et contenant 1 à 2 atomes d'oxygène et/ou de soufre, éventuellement substitué une à six fois identiques ou différentes par du fluor, du chlore, du brome, un radical alkyle linéaire ou ramifié ayant 1 à 4 atomes de carbone et/ou un radical halogénalkyle linéaire ou ramifié ayant 1 à 4 atomes de carbone et 1 à 9 atomes de fluor, de chlore et/ou de brome.

3. Composés de formule (I) suivant la revendication 1, dans lesquels
R¹ représente de l'hydrogène, un groupe alkyle linéaire ou ramifié ayant 1 à 4 atomes de carbone ou un groupe alkoxy linéaire ou ramifié ayant 1 à 4 atomes de carbone,
R² représente de l'hydrogène, un groupe alkyle linéaire ou ramifié ayant 1 à 4 atomes de carbone ou un groupe alkoxy linéaire ou ramifié ayant 1 à 4 atomes de carbone,
R³ est de l'hydrogène, un groupe alkyle linéaire ou ramifié ayant 1 à 4 atomes de carbone ou un groupe alkoxy linéaire ou ramifié ayant 1 à 4 atomes de carbone,
R⁴ représente du fluor, du chlore, du brome, un groupe cyano, nitro, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, trifluorométhyle, trifluorométhoxy, difluorométhoxy, trifluorométhylthio, difluorochlorométhylthio, trifluorométhylsulfinyle, trifluorométhylsulfonyle, diméthylamino, diéthylamino, acétyle, acétoxy, méthylsulfonyloxy, éthylsulfonyloxy, méthoxycarbonyle, éthoxycarbonyle, hydroximinométhyle, hydroximinoéthyle, méthoximinométhyle, méthoximinoéthyle, éthoximinométhyle, éthoximinoéthyle, cyclopropyle, cyclopentyle, cyclohexyle, 1-pyrrolidinyle, 1-pipéridinyle, 1-perhydroazépinyle, 4-morpholinyle ou un reste phényle, phénoxy, benzyle, benzyloxy, phényléthyle ou phényléthyloxy, chacun des six restes mentionnés en dernier lieu pouvant être substitué dans la partie phényle une à trois fois identiques ou différentes par du fluor, du chlore, du brome, un radical méthyle, éthyle, méthoxy, éthoxy, trifluorométhyle et/ou trifluorométhoxy,
R⁵ représente du fluor, du chlore, du brome, un groupe cyano, nitro, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, trifluorométhyle, trifluorométhoxy, difluorométhoxy, trifluorométhylthio, difluorochlorométhylthio, trifluorométhylsulfinyle, trifluorométhylsulfonyle, diméthylamino, diéthylamino, acétyle, acétoxy, méthylsulfonyloxy, éthylsulfonyloxy, méthoxycarbonyle, éthoxycarbonyle, hydroximinométhyle, hydroximinoéthyle, cyclopropyle, cyclopentyle, cyclohexyle, 1-pyrrolidinyle, 1-pipéridinyle, l-perhydro-azépinyle, 4-morpholinyle ou un reste phényle, phénoxy, benzyle, benzyloxy, phényléthyle ou phényléthyloxy, chacun des six restes mentionnés en dernier lieu pouvant être substitué dans la partie phényle une à trois fois identiques ou différentes par du fluor, du chlore, du brome, un radical méthyle, éthyle, méthoxy, éthoxy, trifluorométhyle et/ou trifluorométhoxy,
m représente les nombres 0, 1 ou 2, R⁴ pouvant représenter des restes identiques ou différents lorsque m est égal à 2,
n représente les nombres 1 ou 2, R⁵ pouvant représenter des restes identiques ou différents lorsque n est égal à 2,
A est un groupement de formule -CH₂-O-, -O-CH₂-. -CH₂-S- ou -S-CH₂-,
R⁶ représente de l'hydrogène, du fluor, du chlore, du brome, un groupe cyano, nitro, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, trifluorométhyle, trifluorométhoxy, difluorométhoxy, trifluorométhylthio, difluorochlorométhylthio, trifluorométhylsulfinyle, trifluorométhylsulfonyle, diméthylamino, diéthylamino, acétyle, acétoxy, méthylsulfonyloxy, éthylsulfonyloxy, méthoxycarbonyle, éthoxycarbonyle, hydroximinométhyle, hydroximinoéthyle, cyclopropyle, cyclopentyle, cyclohexyle, 1-pyrrolidinyle, 1-pipéridinyle, l-perhydro-azépinyle, 4-morpholinyle ou un reste phényle, phénoxy, benzyle, benzyloxy, phényléthyle ou phényléthyloxy, chacun des six restes mentionnés en dernier lieu pouvant être substitué dans la partie phényle une à trois fois identiques ou différentes par du fluor, du chlore, du brome, un radical méthyle, éthyle, méthoxy, éthoxy, trifluorométhyle et/ou trifluorométhoxy,
R⁷ représente un groupe halogénalkoxy linéaire ou ramifié ayant chacun 1 à 3 atomes de carbone et 1 à 7 atomes de fluor, de chlore et/ou de brome, ou un groupe halogénalkylthio linéaire ou ramifié ayant 1 à 3 atomes de carbone et 1 à 7 atomes de fluor, de chlore et/ou de brome,
en outre,
R⁶ et R⁷ représentent ensemble un reste alcanediyle à deux liaisons ayant 1 à 3 atomes de carbone et contenant 1 ou 2 atomes d'oxygène et/ou de soufre, éventuellement substitué une à quatre fois identiques ou différentes par du fluor, du chlore, du brome, un radical méthyle, éthyle et/ou trifluorométhyle.

4. Procédé de production de 2-oximino-2-phénylacétamides de formule 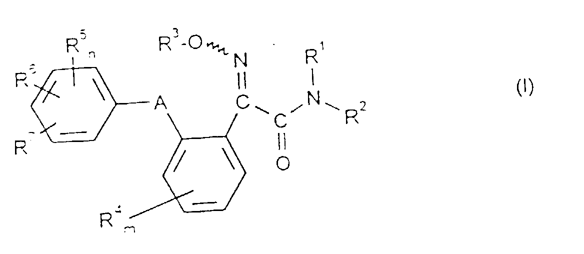 dans laquelle
R¹, R², R³, R⁴, R⁵, R⁶, R⁷, m, n et A ont les définitions indiquées dans la revendication 1,
caractérisé en ce qu'on fait réagir un ester d'acide 2-oximino-2-phénylacétique de formule 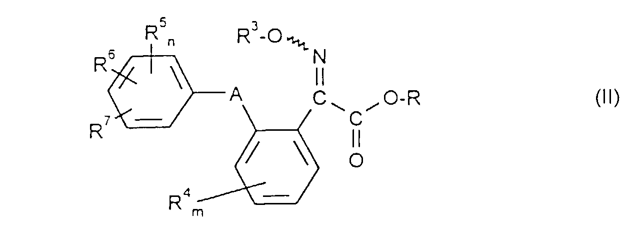 dans laquelle
R³, R⁴, R⁵, R⁶, R⁷, A, m et n ont les définitions indiquées ci-dessus
et
R est un groupe alkyle,
avec l'ammoniac ou des amines de formule 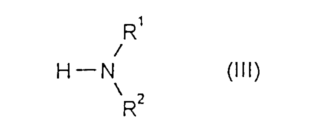 dans laquelle
R¹ et R² ont les définitions indiquées ci-dessus,
le cas échéant en présence d'un diluant et en la présence éventuelle d'un accepteur d'acide.

5. Compositions pesticides, caractérisées par une teneur en au moins un 2-oximino-2-phénylacétamide de formule (I) suivant la revendication 1.

6. Utilisation de 2-oximino-2-phénylacétamides de formule (I) suivant la revendication 1 pour combattre des parasites.

7. Procédé pour combattre des parasites, caractérisé en ce qu'on applique des 2-oximino-2-phénylacétamides de formule (I) suivant la revendication 1 sur les parasites et/ou sur leur milieu.

8. Procédé de préparation de compositions pesticides, caractérisé en ce qu'on mélange des 2-oximino-2-phénylacétamides de formule (I) suivant la revendication 1 avec des diluants et/ou des agents tensio-actifs.
